# EUROPEAN PATENT APPLICATION

(11) **EP 4 086 241 A1**
(43) Date of publication of application: **09.11.2022**
(21) Application number: 20909307.9
(22) Date of filing: 29.12.2020
(51) Int. Cl.: C07D 215/02, C07D 263/54, A61K 31/47, A61K 31/423, A61K 31/136, A61P 27/02, A61Q 19/00

(54) **TRICYCLIC COMPOUND, AND PREPARATION METHOD THEREFOR AND MEDICAL USE THEREOF**

(30) Priority: 30.12.2019 CN 201911391888
(71) Applicant: The National Institutes of Pharmaceutical R&D Co., Ltd, Changping District Beijing 102206 (CN)
(72) Inventor: YIN, Huijun, Beijing 102206 (CN); YAN, Xu, Beijing 102206 (CN); LIU, Guobiao, Beijing 102206 (CN); SHI, Jianxin, Beijing 102206 (CN); CHEN, Shizhu, Beijing 102206 (CN); REN, Siyang, Beijing 102206 (CN); FENG, Yadong, Beijing 102206 (CN)
(74) Representative: EP&C
(86) International application number: PCT/CN2020/140710
(87) International publication number: WO 2021/136244

(57) **Abstract**

Disclosed are a tricyclic compound as represented by general formula (I), a pharmaceutical composition containing same, and a preparation method therefor and the medical use thereof. The compound can be used for preventing and treating a variety of diseases caused by toxic aldehydes.

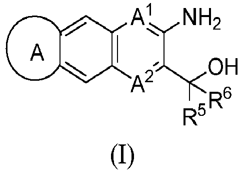

## Description

### TECHNICAL FIELD

The present application relates to a novel tricyclic compound, a pharmaceutical composition comprising the same, a method for preparing the same, and a use thereof in preventing and treating various diseases caused by toxic aldehydes.

### BACKGROUND

The active carbonyl-containing compounds such as aldehydes generally refer to any highly active electrophilic compounds containing one or more carbonyl groups. They originate from a wide range of sources, and participate in various life behaviors and physiological activities. Aldehydes are everywhere in the environment from air, water and soil to food, daily necessities and indoor living places (Koren and Bisesi, CRC press, 2002). Natural sources of aldehyde include plants, animals, microorganisms and natural life processes (O'Brien et al., Critical reviews in toxicology, 2005, 35(7): 609-662). Among them, plants are the main source of aldehyde, for example, vanillin, cinnamaldehyde, benzaldehyde, citral and crotonaldehyde are usually plant-derived aldehydes (Koren and Bisesi, CRC press, 2002; Feron et al., Mutation Research/Genetic Toxicology, 1991, 259(3-4): 363-385). Artificial sources mainly include car exhaust, burning of substances, smoking of cigarettes, overcooking of certain foods, industrial emissions and the like, and aldehydes originated from these sources include formaldehyde, acetaldehyde, benzaldehyde, propionaldehyde, acrolein, glyoxal, glutaraldehyde, crotonaldehyde, *m*-tolualdehyde, 2,5-dimethylbenzaldehyde, 3-hydroxybenzaldehyde and the like (Koren and Bisesi, CRC press, 2002; O'Brien et al., Critical reviews in toxicology, 2005, 35(7): 609-662; Marnett et al., Health Effects Institute. National Academy, 1988). In addition to these sources, aldehydes are also produced in the body through various physiological processes, for example, aldehydes are produced in endogenous pathways such as lipid peroxidation, biotransformation of drug and food, intermediates in biosynthetic and catabolic pathways, and final products of enzymatic reactions (Feron et al., Mutation Research/Genetic Toxicology, 1991, 259(3-4): 363-385; O'Brien et al., Critical reviews in toxicology, 2005, 35(7): 609-662). Generally, the aldehydes produced through these metabolic pathways include 4-hydroxy-2-nonenal, nonenal, acetaldehyde, acrolein, glutamic acid γ-semialdehyde, malondialdehyde, glyoxal, methylglyoxal, glycolaldehyde, glyceraldehyde, lactaldehyde and the like (Esterbauer et al., Free radical Biology and medicine, 1991, 11(1): 81-128; Voulgaridou et al., Mutation Research/Fundamental and Molecular Mechanisms of Mutagenesis, 2011, 711(1-2): 13-27; Nelson et al., Current opinion in pharmacology, 2017, 33: 56-63; Barrera et al., Antioxidants & redox signaling, 2015, 22(18): 1681-1702).

Most active carbonyl compounds have high reactivity, and can act as electrophiles to undergo addition reactions with nucleophiles. This electrophilic-nucleophilic reaction and the resulting adducts are the main reasons for the physiological functions and toxicity of aldehydes. The overexpression and elimination disorder involve in, but not limited to, mitochondrial disruption, membrane damage, endoplasmic reticulum stress, activation of inflammatory mediators, immune dysfunction and the like (Moghe et al., Toxicological Sciences, 2015, 143(2): 242-255). For example, 4-hydroxy-2-nonenal and nonenal produced during lipid peroxidation can easily react with low molecular weight compounds or macromolecules (such as proteins and DNA) in physiological system, and there are reports demonstrate that adduct-related diseases include cancer, neurodegenerative disease, chronic inflammatory disease, autoimmune disease and the like (Barrera et al., Antioxidants & redox signaling, 2015, 22(18): 1681-1702). Aldehydes are in equilibrium in normal physiological system, when this equilibrium is disrupted, the production of adducts of aldehydes with proteins, nucleic acids and phospholipids and the production and accumulation of advanced lipid oxidation end products (ALE) and advanced glycation end products (AGE) will occur (Singh et al., The Korean Journal of Physiology & Pharmacology, 2014, 18(1): 1-14). For example, methylglyoxal, a by-product of glucose metabolism, can be adducted with arginine (Arg) and lysine (Lys) on proteins to form imidazolidinone and carboxyethyllysine, and glyoxal, a product of the autoxidation of carbohydrate and ascorbate, can form carboxymethyllysine with Lys. The dicarbonyl group can directly and specifically react with Arg residue, which has the highest probability of being located in the functional site of protein. Therefore, Arg modification will result in the loss of side chain guanidino groups and important functional Arg residues. Under normal conditions, the protein carbonylation level is usually 1 to 5%, but with aging and disease, the level will increase. Proteins modified by methylglyoxal and glyoxal will be recognized by the body as misfolded proteins and directly degraded by the proteasome (Thornalley et al., Nucleic acids research, 2010, 38(16): 5432-5442).

Carbonyl stress caused by reactive carbonyl compound can lead to nonspecific modification of protein and genetic material, resulting in cytotoxicity. Carbonyl stress can mediate mitochondrial protein dysfunction and increased reactive oxygen species formation (Yao and Brownlee, Diabetes, 2010, 59(1): 249-255), expression of crystallin and inflammatory protein (Yao and Brownlee, Diabetes, 2010, 59(1): 249-255; Ahmed et al., Diabetologia, 2005, 48(8): 1590-1603), lipid-related lipoprotein abnormalities (Rabbani et al., Diabetologia. 233 SPRING ST, NEW YORK, NY 10013 USA: SPRINGER, 2009, 52: S498-S499), activation of mitochondrial apoptosis pathway (Chan et al., Journal of cellular biochemistry, 2007, 100(4): 1056-1069), and cell detachment from the extracellular matrix and apoptosis (Dobler et al., Diabetes, 2006, 55(7): 1961-1969). In addition to carbonyl stress, the protein damage caused by reactive carbonyl species can also modify the amino acid side chain residue of specific protein by enhancing the oxidative stress formed by the generation of reactive oxygen species, resulting in protein carbonylation and changes in protein activity and function. The carbonylation of protein reversibly or irreversibly changes the spatial conformation of polypeptide chain, and partially or completely inhibits protein activity, thereby causing cell dysfunction and tissue damage.

Moreover, highly reactive carbonyl compounds can also lead to reduced enzymatic activity through modification of the enzymatic structure. For example, the reductase activity of hepatic mitochondrial cytochrome C is negatively correlated with the carbonylation of protein, indicating that the oxidation of protein can significantly reduce enzyme activity (Bruno et al., Journal of proteome research, 2009, 8(4): 2070-2078). Active carbonyl species increase the oxidation and carbonylation of protein by inhibiting key cellular enzymes such as glutathione reductase and peroxidase (Shangari et al., Biochemical pharmacology, 2006, 71(11): 1610-1618). In human and mouse, the carbonylation of adipocyte fatty acid-binding protein-4 and epidermal fatty acid-binding protein-5 results in reduced ability to bind fatty acids, reduced lipolysis, and obesity. Fatty acid transporter comprises two cysteine (Cys) residues, which are also susceptible to carbonylation (Febbraio et al., Cellular Lipid Binding Proteins. Springer, Boston, MA, 2002: 193-197). Fatty acid-binding protein prevents the lipotoxicity of long-chain fatty acid by binding or cross-linking with oxidized reactive long-chain fatty acid, and hepatic fatty acid-binding protein depletion will convert nonalcoholic fatty liver to nonalcoholic steatohepatitis (Charlton et al., Hepatology, 2009, 49(4): 1375-1384).

Compared with reactive oxygen species, reactive carbonyl species derived from lipid and carbohydrate are more stable, and can integrate into or even escape cellular degradation and attack targets once formed. Therefore, these soluble active mediators and AGE precursors are not only cytotoxic, but also act as the mediator and communicator of oxidative stress and tissue damage, and play the role of "cytotoxic second messengers", which are risk factors for various diseases throughout the body. Studies have reported that human diseases related to active carbonyl species include cardiovascular diseases, such as atherosclerosis, hypertension, cardiopulmonary dysfunction and the like; respiratory system diseases, such as airway neuroinflammation, chronic obstructive pulmonary disease, respiratory allergy, asthma and the like; neurodegenerative diseases, such as Alzheimer's disease; diabetes and its complications; ocular diseases, such as dry eye, cataract, retinopathy, keratoconus, Fukker's endothelial dystrophy, retinitis pigmentosa, glaucoma, allergic conjunctivitis, uveitis; skin diseases, such as psoriasis, psoriasis, contact dermatitis, atopic dermatitis, acne, Sjögren's syndrome and the like; autoimmune diseases, such as lupus erythematosus and the like; neurological diseases, such as autism, central nervous system toxicity, amyotrophic lateral sclerosis and the like; digestive system diseases, such as neurohepatitis, alcoholic liver disease, non-alcoholic fatty liver disease, ulcerative colitis and the like; and obesity, cancer, and aging-related diseases. Therefore, reduction or elimination of active carbonyl species can improve or alleviate these pathological symptoms.

Treatment of various conditions related to reactive carbonyl species by administration of small molecule therapeutics that act as capture agents for reactive carbonyl species (such as malondialdehyde, 4-hydroxynonenal) has not been proposed in the art. Therefore, there is a need to treat, prevent diseases or conditions in which active carbonyl toxicity is involved in the pathogenesis, and/or reduce the risk of such diseases or conditions. The present invention addresses such need.

### SUMMARY OF THE INVENTION

After deep research, the inventors have designed and synthesized a series of tricyclic compounds, which can be used to prevent and treat a disease related to active carbonyl compound.

Therefore, an object of the present invention is to provide a compound of formula (I): 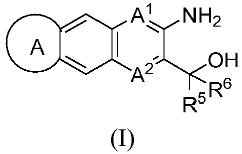
or a mesomer, racemate, enantiomer, diastereomer thereof, or mixture thereof, or a prodrug thereof, or a pharmaceutically acceptable salt thereof,
wherein:
   A¹ is selected from the group consisting of N and CR¹;
   A² is selected from the group consisting of N and CR²;
   ring A is selected from the group consisting of 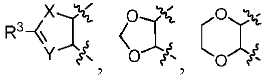 and 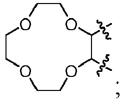 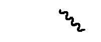 refers to the site attached to the phenyl moiety;
   R¹ and R² are each independently selected from the group consisting of hydrogen, halogen, amino, nitro, cyano, hydroxy, thiol, oxo, alkyl, alkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
   R³ is selected from the group consisting of hydrogen, halogen, amino, nitro, cyano, hydroxy, thiol, oxo, alkyl, alkoxy, haloalkyl, haloalkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
   X and Y are each independently selected from the group consisting of O and NR⁴;
   R⁴ is selected from the group consisting of hydrogen, halogen, amino, nitro, cyano, hydroxy, thiol, oxo, alkyl, alkoxy, haloalkyl, haloalkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
   R⁵ and R⁶ are each independently selected from the group consisting of hydrogen, halogen, amino, cyano, hydroxy, thiol, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
   or, R⁵ and R⁶ together with the carbon atom to which they are attached form a cycloalkyl or heterocyclyl, wherein the cycloalkyl or heterocyclyl is optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
   R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
   or, R^{a} and R^{b} together with the nitrogen atom to which they are attached form a nitrogen-containing heterocyclyl, wherein the nitrogen-containing heterocyclyl is optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl and heteroaryl;
   m is an integer from 0 to 2.

In a preferred embodiment, the compound of formula (I) or the mesomer, racemate, enantiomer, diastereomer thereof, or mixture thereof, or the prodrug thereof, or the pharmaceutically acceptable salt thereof according to the present invention,
wherein:
A¹ is selected from CR¹; and
A² is selected from the group consisting of N and CR²;
R¹ and R² are each independently selected from the group consisting of hydrogen, halogen, C₁-C₆ alkyl, C₁-C₆ alkoxy, C₃-C₆ cycloalkyl and 5- to 6-membered heterocyclyl, and preferably hydrogen and C₁-C₆ alkyl.

In another preferred embodiment, the compound of formula (I) or the mesomer, racemate, enantiomer, diastereomer thereof, or mixture thereof, or the prodrug thereof, or the pharmaceutically acceptable salt thereof according to the present invention,
wherein,
ring A is selected from the group consisting of 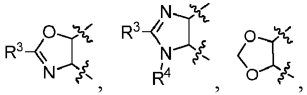 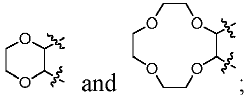
R³ is selected from the group consisting of hydrogen, halogen, amino, hydroxy, thiol, C₁-C₆ alkyl, C₁-C₆ alkoxy, C₁-C₆ haloalkyl, C₁-C₆ haloalkoxy, C₃-C₆ cycloalkyl, 5- to 6-membered heterocyclyl, C₆-C₁₀ aryl and 5- to 10-membered heteroaryl, and preferably hydrogen, C₁-C₆ alkyl, C₁-C₆ haloalkyl, C₃-C₆ cycloalkyl and 5- to 6-membered heterocyclyl;
R⁴ is selected from the group consisting of hydrogen, halogen, amino, hydroxy, thiol, C₁-C₆ alkyl, C₁-C₆ alkoxy, C₁-C₆ haloalkyl, C₁-C₆ haloalkoxy, C₃-C₆ cycloalkyl, 5- to 6-membered heterocyclyl, C₆-C₁₀ aryl and 5- to 10-membered heteroaryl, and preferably hydrogen, C₁-C₆ alkyl and C₁-C₆ haloalkyl.

In another preferred embodiment, the compound of formula (I) or the mesomer, racemate, enantiomer, diastereomer thereof, or mixture thereof, or the prodrug thereof, or the pharmaceutically acceptable salt thereof according to the present invention,
wherein,
R⁵ and R⁶ are each independently selected from the group consisting of hydrogen, halogen, hydroxy, thiol, C₁-C₆ alkyl and C₃-C₆ cycloalkyl, and preferably hydroxy, thiol and C₁-C₆ alkyl, wherein the C₁-C₆ alkyl is optionally further substituted by one or more substituents selected from halogen;
or, R⁵ and R⁶ together with the carbon atom to which they are attached form a C₃-C₆ cycloalkyl, wherein the C₃-C₆ cycloalkyl is optionally further substituted by one or more substituents selected from the group consisting of halogen, hydroxy, thiol and C₁-C₆ alkyl.

Typical compounds of the present invention include, but are not limited to:

| Example No. | Structure and name |
|---|---|
| 1 | 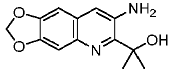 |
| | 2-(7-Amino-[1,3]dioxolo[4,5-*g*]quinolin-6-ylpropan-2-ol |
| 2 | 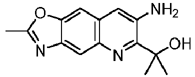 |
| | 2-(7-Amino-2-methyloxazolo[5,4-*g*]quinolin-6-yl)propan-2-ol |
| 3 | 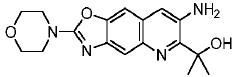 |
| | 2-(7-Amino-2-morpholinooxazolo[5,4-*g*]quinolin-6-yl)propan-2 -ol |
| 4 | 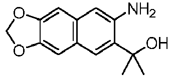 |
| | 2-(7-Aminonaphtho[2,3-*d*][1,3]dioxol-6-yl)propan-2-ol |
| 5 | 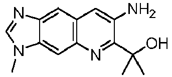 |
| | 2-(7-Amino-3-methyl-3*H*-imidazo[4,5-*g*]quinolin-6-yl)propan-2 -ol |
| 6 | 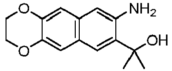 |
| | 2-(8-Amino-1,3-dihydronaphtho[2,3-*b*][1,4]dioxin-7-yl)propan-2-ol |
| 7 | 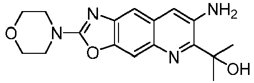 |
| | 2-(7-Amino-2-morpholinooxazolo[4,5-*g*]quinolin-6-yl)propan-2 -ol |
| 8 | 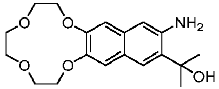 |
| | 2-(14-Amino-2,3,5,6,8,9-hexahydronaphtho[2,3-*b*][1,4,7,10]tetr aoxacyclododecin-13-yl)propan-2-ol |
| 9 | 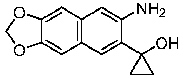 |
| | 1-(7-Aminonaphtho[2,3-*d*][1,3]dioxol-6-yl)cyclopropan-1-ol |

or a mesomer, racemate, enantiomer, diastereomer thereof, or mixture thereof, or a prodrug thereof, or a pharmaceutically acceptable salt thereof.

The present invention further provides a method for preparing the compound of formula (I) or the mesomer, racemate, enantiomer, diastereomer thereof, or mixture thereof, or the prodrug thereof, or the pharmaceutically acceptable salt thereof according to the present invention, comprising the following step of: 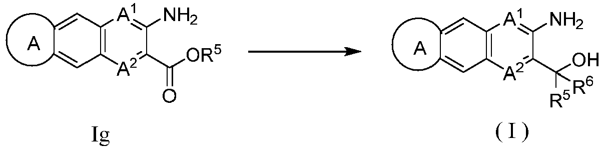
reacting compound Ig with an alkyl Grignard reagent to obtain the compound of formula (I), wherein the alkyl Grignard reagent is preferably methylmagnesium chloride or methylmagnesium bromide; the reaction is preferably carried out in a solvent, the solvent is preferably anhydrous tetrahydrofuran;
wherein A¹, A², ring A, R⁵ and R⁶ are as defined in formula (I).

The present invention further provides a pharmaceutical composition comprising the compound of formula (I) or the mesomer, racemate, enantiomer, diastereomer thereof, or mixture thereof, or the prodrug thereof, or the pharmaceutically acceptable salt thereof according to the present invention, and a pharmaceutically acceptable carrier.

The present invention further provides a use of the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention in the preparation of a toxic aldehyde capture agent.

The present invention further provides a use of the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention in the preparation of a medicament for the prevention and/or treatment of diseases related to active carbonyl compound; the disease is preferably ocular disease, skin disease, autoimmune disease, digestive system disease, cardiovascular disease, respiratory system disease, neurodegenerative disease, obesity, cancer, and aging-related disease; and the ocular disease is preferably noninfectious uveitis, allergic conjunctivitis and dry eye.

In another aspect, the present invention provides the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention, for use as a toxic aldehyde capture agent.

In another aspect, the present invention provides the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention, for use as a medicament; preferably, the medicament is used for preventing and/or treating a disease related to active carbonyl compound; the disease is preferably ocular disease, skin disease, autoimmune disease, digestive system disease, cardiovascular disease, respiratory system disease, neurodegenerative disease, obesity, cancer, and aging-related disease; specifically, the medicament is used for preventing and/or treating an ocular disease such as noninfectious uveitis, allergic conjunctivitis and dry eye.

In another aspect, the present invention provides the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention, for use in the prevention and/or treatment of diseases, the disease is selected from the group consisting of ocular disease, skin disease, autoimmune disease, digestive system disease, cardiovascular disease, respiratory system disease, neurodegenerative disease, obesity, cancer, and aging-related disease; and the ocular disease is preferably noninfectious uveitis, allergic conjunctivitis and dry eye.

A method for preventing and/or treating a disease related to active carbonyl compound, comprising a step of administration of a preventively or therapeutically effective dose of the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention to a patient in need thereof; the disease is selected from the group consisting of ocular disease, skin disease, autoimmune disease, digestive system disease, cardiovascular disease, respiratory system disease, neurodegenerative disease, obesity, cancer, and aging-related disease; and the ocular disease is preferably noninfectious uveitis, allergic conjunctivitis and dry eye.

The prodrug of the present invention is a derivatives of the compound of formula (I), which may have weak or even no activity *per se,* but can be converted to the corresponding biologically active forms under physiological conditions (for example by metabolism, solvolysis or other ways) upon administration.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such composition can contain one or more ingredients selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the preparation of tablets. These excipients may be inert excipients, such as calcium carbonate, sodium carbonate, lactose, calcium phosphate or sodium phosphate; granulating and disintegrating agents, such as microcrystalline cellulose, cross-linked sodium carboxylmethyl cellulose, corn starch or alginic acid; binders, such as starch, gelatin, polyvinylpyrrolidone or acacia; and lubricants, such as magnesium stearate, stearic acid or talc. The tablet may be uncoated or coated by means of known techniques, which can mask drug taste or delay the disintegration and absorption of the active ingredient in the gastrointestinal tract, thereby providing sustained release over an extended period. For example, a water-soluble taste masking material can be used, such as hydroxypropyl methylcellulose or hydroxypropyl cellulose, or an extended-release material can be used, such as ethyl cellulose, cellulose acetate butyrate.

An oral formulation can also be provided as hard gelatin capsules in which the active ingredient is mixed with an inert solid diluent, such as calcium carbonate, calcium phosphate or kaolin, or as soft gelatin capsules in which the active ingredient is mixed with a water-soluble carrier such as polyethylene glycol or an oil medium such as peanut oil, liquid paraffin or olive oil.

An aqueous suspension contains the active ingredient in admixture with an excipient suitable for the preparation of aqueous suspension. Such excipient is a suspending agent, such as sodium carboxylmethylcellulose, methylcellulose, hydroxypropylmethylcellulose, sodium alginate, polyvinylpyrrolidone and acacia; a dispersant or humectant, which can be a naturally occurring phosphatide such as lecithin, or a condensation product of an alkylene oxide with fatty acid such as polyoxyethylene stearate, or a condensation product of ethylene oxide with a long chain aliphatic alcohol such as heptadecaethyleneoxy cetanol, or a condensation product of ethylene oxide with part esters derived from fatty acids and hexitols such as polyoxyethylene sorbitol monooleate, or a condensation product of ethylene oxide with partial esters derived from fatty acids and hexitol anhydrides such as polyoxyethylene sorbitan monooleate. The aqueous suspension can also contain one or more preservatives, such as ethylparaben or *n*-propylparaben, one or more colorants, one or more flavoring agents, and one or more sweeteners such as sucrose, saccharin or aspartame.

An oil suspension can be formulated by suspending the active ingredient in a vegetable oil such as peanut oil, olive oil, sesame oil or coconut oil, or in a mineral oil such as liquid paraffin. The oil suspension can contain a thickener, such as beeswax, hard paraffin or cetyl alcohol. The above sweetener and flavoring agent can be added to provide a palatable formulation. These compositions can be preserved by adding an antioxidant, such as butylated hydroxyanisole or *α*-tocopherol.

A dispersible powder or granule suitable for the preparation of an aqueous suspension can provide active ingredient by adding water and dispersants or wetting agents, suspending agent or one or more preservatives. Suitable dispersants, wetting agents and suspending agents are as described above. Additional excipients, such as sweetening agents, flavoring agents and coloring agents, can also be added. These compositions are preserved by adding an antioxidant such as ascorbic acid.

The pharmaceutical composition of the present invention can also be in the form of an oil-in-water emulsion. The oil phase can be a vegetable oil such as olive oil or peanut oil, or a mineral oil such as liquid paraffin or a mixture thereof. Suitable emulsifying agent can be naturally occurring phosphatides, such as soy bean lecithin, and esters or partial esters derived from fatty acids and hexitol anhydrides such as sorbitan monooleate, and condensation products of said partial esters with ethylene oxide such as polyoxyethylene sorbitol monooleate. The emulsion can also contain a sweetener, flavoring agent, preservative and antioxidant. Syrup and elixir can be formulated with a sweetener, such as glycerol, propylene glycol, sorbitol or sucrose. Such formulations can also contain a moderator, a preservative, a colorant and an antioxidant.

The pharmaceutical composition of the present invention can be in the form of a sterile injectable aqueous solution. The acceptable medium or solvent that can be used include water, Ringer's solution and isotonic sodium chloride solution. The sterile injectable formulation can be a sterile injectable oil-in-water microemulsion in which the active ingredient is dissolved in the oil phase. For example, the active ingredient can be firstly dissolved in a mixture of soybean oil and lecithin, the oil solution is then introduced into a mixture of water and glycerol and processed to form a microemulsion. The injectable solution or microemulsion can be introduced into a patient's bloodstream by local bolus injection. Alternatively, it may be advantageous to administer the solution or microemulsion in such a way as to maintain a constant circulation concentration of the compound of the present invention. In order to maintain such a constant concentration, a continuous intravenous delivery device can be utilized.

The pharmaceutical composition of the present invention can be in the form of a sterile injectable aqueous or oily suspension for intramuscular and subcutaneous administration. Such a suspension can be formulated with suitable dispersants, wetting agents and suspending agents as described above according to known techniques. The sterile injectable formulation can also be a sterile injectable solution or suspension prepared in a nontoxic parenterally acceptable diluent or solvent, such as a solution prepared in 1,3-butanediol. Moreover, sterile fixed oils can easily be used as a solvent or suspending medium. For this purpose, any blending fixed oils including synthetic mono- or di-glyceride can be employed. Moreover, fatty acids such as oleic acid can also be employed in the preparation of an injection.

The pharmaceutical composition of the present invention can be in the form for topical administration, for example, a cream, suspension, emulsion, ointment, gel, drop, oil, lotion, film, patch, tape, inhalant, spray. The form for intraocular administration can be a subconjunctival capsule, subfascial capsule, retrobulbar or intravitreal injection, depot injection or implant. The compound administered by these routes can be in the form of solution or suspension. The compound administered by depot injection can contain a pharmaceutically acceptable carrier or excipient. These pharmaceutically acceptable carriers or excipients can be natural or synthetic, biodegradable or non-biodegradable, and facilitate drug release in a controlled manner. The implant for controlled release of compound can be constructed of natural or synthetic, biodegradable or non-biodegradable materials. The carrier is acceptable, since it is compatible with the other components of the composition and is not detrimental to the patient. Some examples of carrier include sugar, such as lactose, dextrose and sucrose; starch, such as corn starch and potato starch; cellulose; and cyclodextrins.

It is well known to those skilled in the art that the dosage of a drug depends on a variety of factors including, but not limited to the following factors: activity of a specific compound, age of the patient, body weight of the patient, general health of the patient, behavior of the patient, diet of the patient, administration time, administration route, excretion rate, drug combination and the like. In addition, the optimal treatment, such as treatment mode, daily dose of the compound of the present invention or the type of pharmaceutically acceptable salt thereof can be verified according to the traditional therapeutic regimens.

The present invention may contain a composition comprising the compound of formula (I) or the pharmaceutically acceptable salt, hydrate or solvate as an active ingredient, and a pharmaceutically acceptable carrier or excipient, which is formulated into a clinically acceptable formulation. The derivatives of the present invention can be used in combination with other active ingredients as long as they do not cause other adverse effects such as allergic reactions and the like. The compound of the present invention can be used as the sole active ingredient, and can also be used in combination with other therapeutic agents. A combination therapy is achieved by administering the individual therapeutic components simultaneously, separately or sequentially.

### Definitions

Unless otherwise stated, the terms used in the specification and claims have the meanings described below.

The carbon, hydrogen, oxygen, sulfur, nitrogen and halogen involved in the groups and compounds of the present invention all include their isotopes. That is to say, the carbon, hydrogen, oxygen, sulfur, nitrogen and halogen involved in the groups and compounds of the present invention are optionally further substituted by one or more of their corresponding isotopes. The isotopes of carbon include ¹²C, ¹³C and ¹⁴C; the isotope of hydrogen include protium (H), deuterium (D, also known as heavy hydrogen), tritium (T, also known as superheavy hydrogen); the isotope of oxygen include ¹⁶O, ¹⁷O and ¹⁸O; the isotope of sulfur include ³²S, ³³S, ³⁴S and ³⁶S; the isotopes of nitrogen include ¹⁴N and ¹⁵N; the isotope of fluorine include ¹⁹F; the isotope of chlorine include ³⁵Cl and ³⁷Cl; and the isotope of bromine include ⁷⁹Br and ⁸¹Br.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl*,* 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "alkylene" refers to a linear or branched divalent saturated hydrocarbon group, including -(CH₂)ᵥ- (v is an integer from 1 to 10, and preferably an integer from 1 to 6). Examples of alkylene include, but are not limited to, methylene, ethylene, propylene, butylene and the like. The alkylene group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group. When the number of substituents in an alkylene is 2 or more, the substituents can be fused together to form a cyclic structure.

The term "alkenyl" refers to an alkyl group as defined above consisting of at least two carbon atoms and at least one carbon-carbon double bond, such as ethenyl, 1-propenyl, 2-propenyl, 1-, 2- or 3-butenyl and the like. The alkenyl group can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkenylene" refers to a straight or branched chain divalent alkenyl group, wherein the alkenyl is as defined above.

The term "alkynyl" refers to an alkyl group as defined above consisting of at least two carbon atoms and at least one carbon-carbon triple bond, such as ethynyl, propynyl, butynyl and the like. The alkynyl group can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkynylene" refers to a straight or branched chain divalent alkynyl group, wherein the alkynyl is as defined above.

The term "cycloalkyl" refers to a saturated or partially unsaturated monocyclic or polycyclic hydrocarbon substituent group having 3 to 20 carbon atoms, preferably 3 to 10 carbon atoms, and more preferably 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one commom carbon atom (called a spiro atom), wherein one or more rings can contain one or more double bonds, but none of the rings has a completely conjugated π-electron system. The spiro cycloalkyl is preferably a 5 to 12 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

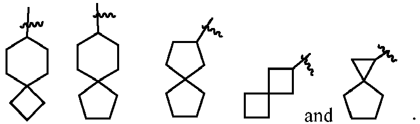

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

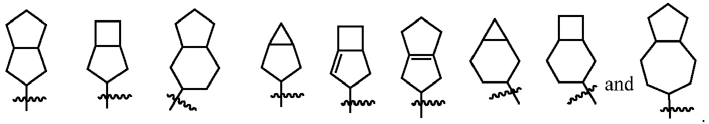

The term "bridged cycloalkyl" refers to a 5 to 20 membered all-carbon polycyclic group, wherein any two rings in the system share two disconnected carbon atoms, one or more rings can have one or more double bonds, but none of the rings has a completely conjugated π-electron system. The bridged cycloalkyl is preferably a 6 to 12 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

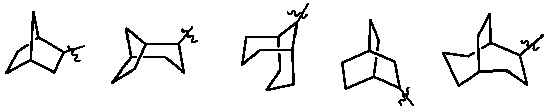

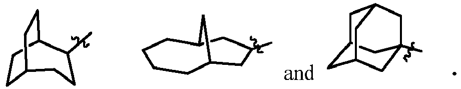

The cycloalkyl ring can be fused to the ring of aryl, heteroaryl or heterocyclyl, wherein the ring linking to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "heterocyclyl" or "heterocycle" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 10 ring atoms wherein 1 to 4 atoms are heteroatoms; more preferably, 3 to 8 ring atoms wherein 1 to 3 atoms are heteroatoms; and most preferably 5 to 7 ring atoms wherein 1 to 2 or 1 to 3 atoms are heteroatoms. Non-limiting examples of monocyclic heterocyclyl include pyrrolidinyl, imidazolyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably, 1,2,5-oxadiazolyl, pyranyl or morpholinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one common atom (called a spiro atom), wherein one or more ring atoms are heteroatoms selected from the group consisting of N, O and S(O)ₘ (wherein m is an integer of 0 to 2), with the remaining ring atoms being carbon atoms, and the rings can contain one or more double bonds, but none of the rings has a completely conjugated π-electron system. The spiro heterocyclyl is preferably a 5 to 12 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

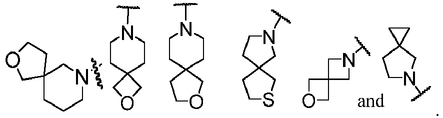

The term "fused heterocyclyl" refers to a 5 to 20 membered polycyclic heterocyclyl group, wherein each ring in the system shares an adjacent pair of atoms with another ring, one or more rings can contain one or more double bonds, but none of the rings has a completely conjugated π-electron system, and one or more ring atoms are heteroatoms selected from the group consisting of N, O and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and preferably a bicyclic or tricyclic fused heterocyclyl, and more preferably a 5-membered/5-membered or 5-membered/6-membered bicyclic fused heterocyclyl. Non-limiting examples of fused heterocyclyl include:

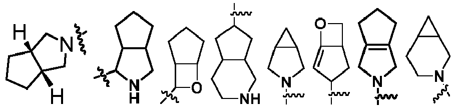

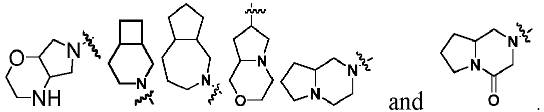

The term "bridged heterocyclyl" refers to a 5 to 14 membered polycyclic heterocyclyl group, wherein any two rings in the system share two disconnected atoms, wherein one or more rings can have one or more double bond(s), but none of the rings has a completely conjugated π-electron system, and one or more ring atoms are heteroatoms selected from the group consisting of N, O and S(O)ₘ (wherein m is an integer of 0 to 2), with the remaining ring atoms being carbon atoms. The bridged heterocyclyl is preferably a 5 to 12 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

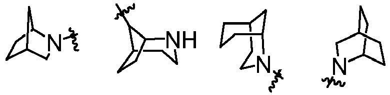

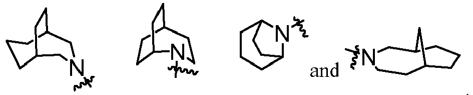

The heterocyclyl ring can be fused to the ring of aryl, heteroaryl or cycloalkyl, wherein the ring bound to the parent structure is heterocyclyl. Non-limiting examples thereof include: 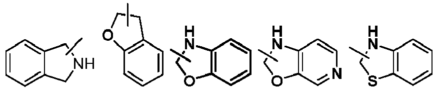 and the like.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "aryl" or "aromatic ring" refers to a 6 to 14 membered all-carbon monocyclic ring or polycyclic fused ring *(i.e.* each ring in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include:

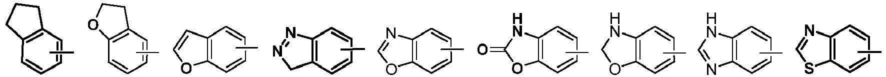

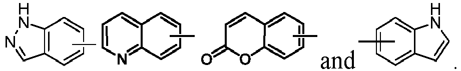

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "heteroaryl" or "heteroaromatic ring" refers to a 5 to 14 membered heteroaromatic system having 1 to 4 heteroatoms selected from the group consisting of O, S and N. The heteroaryl is preferably a 5 to 10 membered heteroaryl having 1 to 3 heteroatom(s), and more preferably a 5 or 6 membered heteroaryl having 1 to 2 heteroatom(s), for example imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, thiazolyl, pyrazolyl, pyrimidinyl or thiazolyl, and more preferably pyrazolyl or thiazolyl. The heteroaryl ring can be fused to the ring of aryl, heterocyclyl or cycloalkyl, wherein the ring bound to the parent structure is heteroaryl ring. Non-limiting examples thereof include:

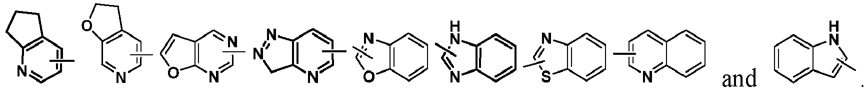

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "alkoxy" refers to an -O-(alkyl) or an -O-(cycloalkyl) group, wherein the alkyl and cycloalkyl are as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "haloalkyl" refers to an alkyl group substituted by one or more halogen(s), wherein the alkyl is as defined above.

The term "haloalkoxy" refers to an alkoxy group substituted by one or more halogen(s), wherein the alkoxy is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to a =O group.

The term "carboxyl" refers to a -C(O)OH group.

The term "thiol" refers to a -SH group.

The term "ester group" refers to a -C(O)O(alkyl) or a -C(O)O(cycloalkyl) group, wherein the alkyl and cycloalkyl are as defined above.

The term "acyl" refers to a compound comprising a -C(O)R group, where R is an alkyl, cycloalkyl, heterocyclyl, aryl or heteroaryl.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the situation of the heterocyclyl being not substituted by an alkyl.

"Substituted" refers to one or more hydrogen atoms in a group, preferably up to 5, and more preferably 1 to 3 hydrogen atoms in a group, are independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

A "carrier" refers to a carrier or diluent that is not appreciably irritating to the organism and that does not reduce the biological activity and properties of the compound.

A "prodrug" refers to a compound that can be converted under physiological conditions or by solvolysis to a compound of the present invention having biological activity. The prodrug of the present invention is prepared by modifying functional groups of the compound of the present invention, and such modification can be removed by conventional process or *in vivo* to give the parent compound.

### DESCRIPTION OF THE DRAWINGS

Figure 1 is a time-varying diagram of the capture reaction of the compounds of some examples of the present invention to nonenal.
Figure 2 is a graph showing the results of the treatment score of the compound of Example 4 of the present invention in the C48/80-induced Wistar rat allergic conjunctivitis animal model.

### DETAILED DESCRIPTION OF THE INVENTION

The compounds of the present invention and their preparation are further illustrated with reference to the following examples. These examples illustrate some methods for preparing or using said compounds. However, it should be understood that these examples are not intended to limit the scope of the present invention. Variations of the present invention now known or to be further developed are considered to fall within the scope of the present invention described and claimed herein.

The compound of the present invention is prepared by using convenient starting materials and general preparation procedures. The present invention provides typical or preferential reaction conditions, such as reaction temperature, duration, solvent, pressure and molar ratio of reactants. However, unless otherwise specified, other reaction conditions can also be adopted. Optimal conditions may vary with the use of specific reactants or solvents, but under normal circumstances, reaction optimization steps and conditions can be determined.

In addition, some protecting groups may be used in the present invention to protect certain functional groups from unnecessary reactions. The protecting groups suitable for various functional groups and their protection or deprotection conditions are well known to those skilled in the art. For example, "Protective Groups in Organic Synthesis" by T. W. Greene and G. M. Wuts (3rd edition, Wiley, New York, 1999 and citations in the book) describes in detail the protection or deprotection of a large number of protective groups.

The separation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The examples described in the present invention can be referred for the specific method of use. Of course, other similar separation and purification methods can also be used. They can be characterized using conventional methods (including physical constants and spectral data).

The structures of the compounds are identified by nuclear magnetic resonance (NMR) and/or mass spectrometry (MS). NMR shifts (δ) are given in 10⁻⁶ (ppm). NMR is determined by a Brukerdps300 machine. The solvents for determination are deuterated-dimethyl sulfoxide (DMSO-*d₆*), deuterated-chloroform (CDCl₃) and deuterated-methanol (CD₃OD), and the internal standard is tetramethylsilane (TMS).

MS is determined by a LC(Waters 2695)/MS(Quattro Premier xE) mass spectrograph (manufacturer: Waters) (Photodiode Array Detector).

Preparative liquid chromatography is conducted on a lc6000 high performance liquid chromatograph (manufacturer: Beijing Chuangxintongheng science and Technology Co., Ltd.).

Qingdao Haiyang Chemical GF254 silica gel plate is used for the thin-layer silica gel chromatography (TLC). The dimension of the silica gel plate used in TLC is 0.20 mm to 0.25 mm, and the dimension of the silica gel plate used in product purification (Prep-TLC) is 0.5 mm.

Qingdao Haiyang Chemical 100 to 200 mesh, 200 to 300 mesh and 300 to 400 mesh silica gel is generally used as a carrier for column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Innochem Science & Technology, Energy Chemical, Shanghai Bide Pharmatech and the like.

Unless otherwise stated, the reactions are carried out under a nitrogen atmosphere.

Argon atmosphere or nitrogen atmosphere means that a reaction flask is equipped with an argon or nitrogen balloon (about 1 L).

The reaction solvent, organic solvent or inert solvent are each expressed as the solvent used that does not participate in the reaction under the described reaction conditions, which includes for example, benzene, toluene, acetonitrile, tetrahydrofuran (THF), dimethylformamide (DMF), chloroform, dichloromethane, ether, methanol, N-methylpyrrolidone (NMP), pyridine and the like. Unless otherwise specified in the examples, a solution means an aqueous solution.

The chemical reaction described in the present invention is generally carried out under normal pressure. The reaction temperature is between -78°C and 200°C. The reaction duration and condition are, for example, between -78°C and 200°C under one atmospheric pressure, and completed within about 1 to 24 hours. If the reaction is conducted overnight, then the reaction duration is generally 16 hours. Unless otherwise specified in the examples, the reaction temperature is room temperature, which is 20°C to 30°C.

The progress of the reaction in the examples is monitored by thin layer chromatography (TLC). The developing systems used in the reactions include: A: dichloromethane and methanol system, B: petroleum ether and ethyl acetate system, C: acetone. The volume ratio of the solvent is adjusted depending on the polarity of the compound.

The eluent systems of column chromatography and the developing systems of TLC for the purification of the compound include: A: dichloromethane and methanol system, B: petroleum ether and ethyl acetate system. The volume ratio of the solvent is adjusted depending on the polarity of the compound, and a small amount of an alkaline or acidic reagent such as triethylamine or trifluoroacetic acid may be added for adjustment.

Unless otherwise defined, all professional and scientific terms used herein have the same meaning as those familiar to those skilled in the art. In addition, any methods and materials similar or equivalent to the content described herein can be applied to the method of the present invention.

### Abbreviations

NMR = nuclear magnetic resonance
Boc = *tert*-butoxycarbonyl
DIEA = diisopropylethylamine
DMF = *N,N*-dimethylformamide
DMSO = dimethyl sulfoxide
DCM = dichloromethane
EA = ethyl acetate
HPLC = high performance liquid chromatography
LC-MS = liquid chromatography-mass spectrometry
tol. = toluene
PE = petroleum ether
TBAF = tetrabutylammonium fluoride
TCDI = N,N'-thiocarbonyldiimidazole
THF = tetrahydrofuran
TMS = trimethylsilyl
TMSOTf = trimethylsilyl trifluoromethanesulfonic acid.

### Example 1: Preparation of 2-(7-amino-[1,3]dioxolo[4,5-g]quinolin-6-yl)propan-2-ol (1)

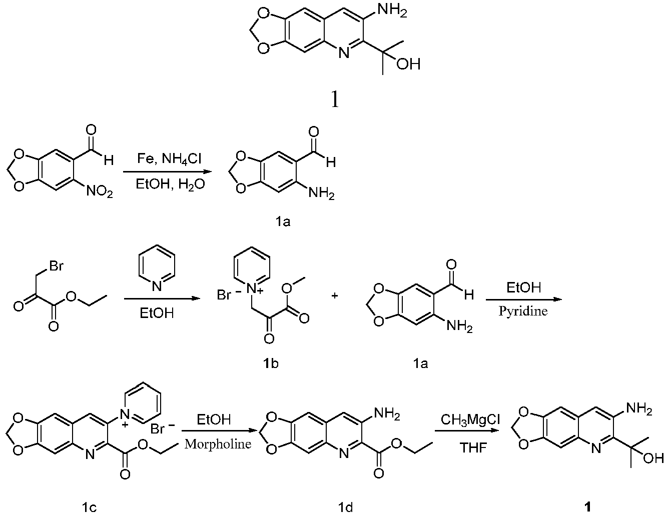

### Step 1: Preparation of 6-aminobenzo[d][1,3]dioxole-5-carbaldehyde (1a)

Iron powder (1.58 g, 28.2 mmol), ammonium chloride (200 mg, 3.60 mmol) were dissolved in a mixed solvent of ethanol (20 ml) and water (2 mL) at room temperature, followed by the addition of 6-nitrobenzo[*d*][1,3]dioxole-5-carbaldehyde (1.00 g, 5.13 mmol). The reaction solution was warmed up to 78°C, and stirred under reflux for 20 hours. The organic phase was extracted with 100 mL of ethyl acetate and 100 mL of water, and the aqueous phase was extracted once with 30 mL of ethyl acetate. The organic phases were combined, washed once with saturated sodium bicarbonate solution, and concentrated under reduced pressure. The residues were purified by silica gel column chromatography (mobile phase: PE/EA = 10:1 to 4:1) to obtain 380 mg of the title compound as a yellow solid, yield: 44.9%.

LC-MS: m/z 165 [M + H]⁺.

### Step 2: Preparation of 1-(3-methoxy-2,3-dioxopropyl)pyridine-1-ammonium bromide (1b)

Pyridine (178 mg, 2.26 mmol) was dissolved in ethanol (10 mL) at room temperature, and then ethyl 3-bromo-2-oxopropanoate (400 mg, 2.05 mmol) was slowly added dropwise under a nitrogen atmosphere. The reaction solution was stirred at 65°C for 2 hours, and used directly in the next step.

### Step 3: Preparation of 1-(6-(ethoxycarbonyl)-[1,3]dioxolo[4,5-g]quinolin-7-yl)pyridine-1-ammonium bromide (1c)

The reaction solution of Step 2 was cooled to 18 to 22°C, to which 6-aminobenzo[*d*][1,3]dioxole-5-carbaldehyde (1a) (304 mg, 1.85 mmol) was added. The reaction solution was warmed up to 80°C and stirred for 18 hours, which was used directly in the next step.

### Step 4: Preparation of ethyl 7-amino-[1,3]dioxolo[4,5-g]quinoline-6-carboxylate (1d)

The reaction solution of Step 3 was cooled to 70°C, followed by the addition of morpholine (446 mg, 5.14 mmol). The mixture was stirred at 80°C for 16 hours. The reaction solution was extracted with 50 mL of water and 50 mL of ethyl acetate, and the aqueous phase was extracted once with 20 mL of ethyl acetate. The organic phases were combined and concentrated under reduced pressure. The residues were purified by silica gel column chromatography (mobile phase: PE/EA = 8:1 to 2:1) to obtain 240 mg of the title compound as a yellow solid, yield: 45.0%.

LC-MS: m/z 260[M + H]⁺.

### Step 5: Preparation of 2-(7-amino-[1,3]dioxolo[4,5-g]quinolin-6-ylpropan-2-ol (1)

Ethyl 7-amino-[1,3]dioxolo[4,5-*g*]quinoline-6-carboxylate (1d) (190 mg, 0.730 mmol) was dissolved in tetrahydrofuran (19 mL) at room temperature. The solution was cooled to 0°C, to which methylmagnesium chloride (1.50 mL, 4.80 mmol) was slowly added dropwise under a nitrogen atmosphere. The reaction solution was stirred at room temperature for 16 hours, and the reaction was quenched with water (10 mL). The reaction solution was extracted with water (30 mL) and ethyl acetate (30 mL), and the aqueous phase was extracted once with 20 mL of ethyl acetate. The organic phases were combined and concentrated under reduced pressure. The residues were purified by preparative liquid chromatography (column model: Daisogei 30 mm x 250 mm, C18, 10 um, 100 Å, mobile phase: acetonitrile/water (0.05% formic acid), gradient: 30%-80%) to obtain 23 mg of the compound as a white solid. The compound was dissolved in 50 mL of ethyl acetate, and the pH was adjusted to about 10 to 11 with 10% aqueous ammonia solution. The organic phase was separated, dried over anhydrous sodium sulfate and filtered. The filtrate was concentrated under reduced pressure to obtain 15 mg of the title compound as a yellow solid, yield: 8.34%.

LC-MS: m/z 247.28 [M + H]⁺.

¹H NMR (300 MHz, DMSO-*d₆*) *δ* 7.13 (s, 1H), 7.06 (s, 1H), 6.99 (s, 1H), 6.06 (s, 2H), 5.62 (s, 2H), 5.60 (s, 1H), 1.57 (s, 6H).

### Example 2: Preparation of 2-(7-amino-2-methyloxazolo[5,4-g]quinolin-6-yl)propan-2-ol (2)

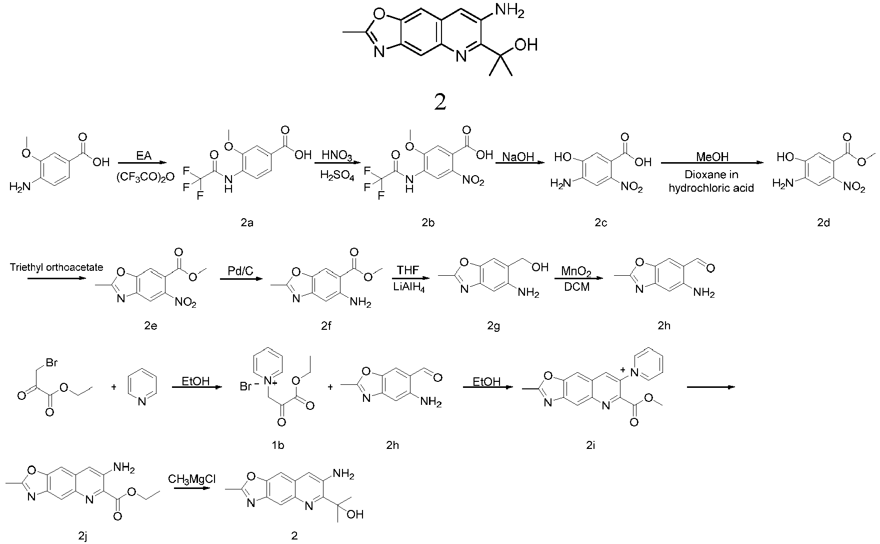

### Step 1: Preparation of 3-methoxy-4-(2,2,2-trifluoroacetamido)benzoic acid (2a)

4-Amino-3-methoxybenzoic acid (5.00 g, 29.9 mmol) was dissolved in ethyl acetate (200 mL) at room temperature, to which a mixed solution of trifluoroacetic anhydride (5 mL, 35.9 mmol) and ethyl acetate (50 mL) was slowly added dropwise. The reaction solution was stirred at room temperature for 16 hours and concentrated under reduced pressure. The residues were dissolved in ethyl acetate, and concentrated twice under reduced pressure to obtain 9.00 g of the crude title compound as a grey solid, which was used directly in the next step without purification.

### Step 2: Preparation of 5-methoxy-2-nitro-4-(2,2,2-trifluoroacetamido)benzoic acid (2b)

3-Methoxy-4-(2,2,2-trifluoroacetamido)benzoic acid (2a) (9.00 g, 34.2 mmol) was dissolved in concentrated sulfuric acid (96 mL) at room temperature, and the solution was cooled in an ice bath under stirring. A mixed solution of concentrated nitric acid (3.30 g) and concentrated sulfuric acid (24 mL) was added dropwise under cooling, and the temperature was kept below 10°C. After completion of the addition, the mixture was further stirred for 10 minutes. The reaction solution was added dropwise into ice water and filtered. The filter cake was rinsed with 200 mL of water, and dried under reduced pressure to obtain 7.20 g of the title compound as a light brown solid, yield: 68.3%.

### Step 3: Preparation of 4-amino-5-hydroxy-2-nitrobenzoic acid (2c)

5-Methoxy-2-nitro-4-(2,2,2-trifluoroacetamido)benzoic acid (2b) (7.00 g, 22.7 mmol) was dissolved in 20% sodium hydroxide solution (50 mL) at room temperature, and the reaction solution was stirred under a nitrogen atmosphere at 100°C for 16 hours. The reaction solution was cooled to room temperature, to which concentrated hydrochloric acid (21 mL) was added dropwise under ice bath cooling. The reaction solution was concentrated under reduced pressure, to which anhydrous ethanol (420 mL) was added. The solution was stirred for 0.5 hour and filtered. The filtrate was concentrated under reduced pressure to obtain 7.20 g of the crude title compound as a brown solid, which was used directly in the next step without purification.

### Step 4: Preparation of methyl 4-amino-5-hydroxy-2-nitrobenzoate (2d)

4-Amino-5-hydroxy-2-nitrobenzoic acid (2c) (7.20 g, 36.4 mmol) was dissolved in MeOH (477 mL) at room temperature, to which dioxane in hydrochloric acid (161 mL) was added and stirred at 64°C for 16 hours. The reaction solution was concentrated under reduced pressure to obtain 6.80 g of the crude title compound as a black oily solid, which was used directly in the next step without purification.

### Step 5: Preparation of methyl 2-methyl-5-nitrobenzo[d]oxazole-6-carboxylate (2e)

Methyl 4-amino-5-hydroxy-2-nitrobenzoate (2d) (5.00 g, 23.6 mmol) was dissolved in triethyl orthoacetate (25 mL) at room temperature, and the solution was stirred at room temperature for 16 hours followed by concentrating under reduced pressure. The residues were purified by silica gel column chromatography (mobile phase: PE/EA = 10:1 to 3:1) to obtain 1.50 g of the title compound as a light yellow solid, yield: 27.0%.

### Step 6: Preparation of methyl 5-amino-2-methylbenzo[d]oxazole-6-carboxylate (2f)

Methyl 2-methyl-5-nitrobenzo[d]oxazole-6-carboxylate (2e) (1.30 g, 5.51 mmol) was dissolved in MeOH (93 mL) at room temperature. Pd/C (195 mg) was added, and the reaction solution was stirred under a hydrogen atmosphere for 16 hours. The reaction solution was filtered through celite, and the filtrate was concentrated under reduced pressure. The residues were purified by silica gel column chromatography (mobile phase: PE/EA = 10:1 to 3:1) to obtain 900 mg of the title compound as a light yellow solid, yield: 79.3%.

### Step 7: Preparation of (5-amino-2-methylbenzo[d]oxazol-6-yl)methanol (2g)

Methyl 5-amino-2-methylbenzo[*d*]oxazole-6-carboxylate (2f) (800 mg, 3.88 mmol) was dissolved in THF (70 mL) at room temperature. Lithium aluminum hydride (5.84 mL) was added dropwise under a nitrogen atmosphere at 0°C, and the reaction solution was stirred for 30 minutes. 10 mL of water was added to the reaction solution to quench the reaction. The reaction solution was extracted with ethyl acetate (80 mL), and the organic phase was concentrated under reduced pressure. The residues were purified by silica gel column chromatography (mobile phase: ethyl acetate) to obtain 350 mg of the title compound as a dark yellow solid, yield: 50.7%.

### Step 8: Preparation of 5-amino-2-methylbenzo[d]oxazole-6-carbaldehyde (2h)

(5-Amino-2-methylbenzo[*d*]oxazol-6-yl)methanol (2g) (340 mg, 1.91 mmol) was dissolved in DCM (10 mL) at room temperature. Manganese dioxide (3.32 g, 38.2 mmol) was added, and the reaction solution was stirred at room temperature for 4 hours. The reaction solution was filtered through celite, and the filtrate was concentrated under reduced pressure. The residues were purified by silica gel column chromatography (mobile phase: ethyl acetate) to obtain 200 mg of the title compound as a dark yellow solid, yield: 59.5%.

### Step 9: Preparation of 1-(3-ethoxy-2,3-dioxopropyl)pyridine-1-ammonium bromide (1b)

Pyridine (110 mg, 14.0 mmol) was dissolved in EtOH (10 mL) at room temperature, and then ethyl 3-bromo-2-oxopropanoate (246 mg, 1.26 mmol) was added under a nitrogen atmosphere. The reaction solution was stirred at 65°C for 2 hours, which was used directly in the next step.

### Step 10: Preparation of 1-((6-methoxycarbonyl)-2-methyloxazolo[5,4-g]quinolin-7-yl)pyridin-1-ammonium bromide (2i)

The reaction solution of Step 9 was cooled to 18 to 22°C, followed by the addition of 5-amino-2-methylbenzo[*d*]oxazole-6-carbaldehyde (2h) (200 mg, 1.14 mmol) and pyridine (230 mg, 2.91 mmol). The reaction solution was stirred under a nitrogen atmosphere at 80°C for 9 hours, which was used directly in the next step.

### Step 11: Preparation of ethyl 7-amino-2-methyloxazolo[5,4-g]quinoline-6-carboxylate (2j)

The reaction solution of Step 10 was cooled to 70°C, followed by the addition of morpholine (275 mg, 3.16 mmol). The reaction solution was warmed up to 80°C, and stirring under a nitrogen atmosphere at 80°C for 4 hours. 50 mL of water was added to the reaction solution, and extracted with 50 mL of dichloromethane. The aqueous phase was extracted twice with 50 mL of dichloromethane. The organic phases were combined, dried over anhydrous sodium sulfate and filtered. The filtrate was concentrated under reduced pressure. The residues were purified by silica gel column chromatography (mobile phase: dichloromethane/ethyl acetate = 10:1 to 2:1) to obtain 80 mg of the title compound as a brown yellow solid, yield: 23.4%.

### Step 12: Preparation of 2-(7-amino-2-methyloxazolo[5,4-g]quinolin-6-yl)propan-2-ol (2)

Methylmagnesium chloride (3N, 0.57 mL, 1.70 mmol) was added dropwise to THF (6 mL) under a nitrogen atmosphere at 0°C. Ethyl 7-amino-2-methyloxazolo[5,4-g]quinoline-6-carboxylate (2j) (70.0 mg, 0.258 mmol) dissolved in THF (4 mL) was added, and the reaction solution was stirred at room temperature for 30 minutes. 5 mL of water was added to quench the reaction. 15 mL of water was added to the reaction solution, and extracted with 20 mL of ethyl acetate. The aqueous phase was extracted twice with 15 mL of ethyl acetate. The organic phases were combined, dried over anhydrous sodium sulfate and filtered. The filtrate was concentrated under reduced pressure. The residues were purified by preparative liquid chromatography (column model: Daisogei 30 mm × 250 mm, C18, 10 um, 100 A, mobile phase: acetonitrile/water, gradient: 10%-60%) to obtain 8.00 mg of the title compound as a light yellow solid, yield: 12.1%.

LC-MS: m/z 258.25 [M+H]⁺.

¹H NMR (300 MHz, DMSO-*d₆*) *δ* 7.89(s,1 H), 7.73 (s,1 H), 7.31 (s,1 H), 5.92 (s,2 H), 5.75 (s,1 H), 2.51 (s, 3H), 1.62 (s, 6H).

### Example 3: Preparation of 2-(7-amino-2-morpholinooxazolo[5,4-g]quinolin-6-yl)propan-2-ol (3)

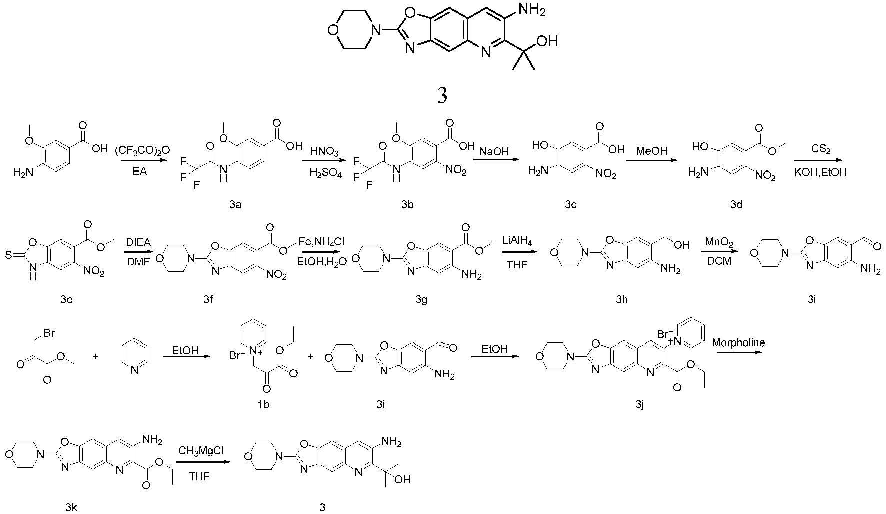

### Step 1: Preparation of 3-methoxy-4-(2,2,2-trifluoroacetamido)benzoic acid (3a)

4-Amino-3-methoxybenzoic acid (5.00 g, 29.9 mmol) was dissolved in ethyl acetate (200 mL) at room temperature, to which a mixed solution of trifluoroacetic anhydride (5 mL, 35.9 mmol) and ethyl acetate (50 mL) was slowly added dropwise. The reaction solution was stirred at room temperature for 16 hours and concentrated under reduced pressure. The residues were dissolved in ethyl acetate, and concentrated twice under reduced pressure to obtain 9.00 g of the crude title compound as a grey solid, which was used directly in the next step without purification.

### Step 2: Preparation of 5-methoxy-2-nitro-4-(2,2,2-trifluoroacetamido)benzoic acid (3b)

3-Methoxy-4-(2,2,2-trifluoroacetamido)benzoic acid (3a) (9.00 g, 34.2 mmol) was dissolved in concentrated sulfuric acid (96 mL) at room temperature, and the solution was cooled in an ice bath under stirring. A mixed solution of concentrated nitric acid (3.30 g) and concentrated sulfuric acid (24 mL) was added dropwise under cooling with keeping the temperature below 10°C. After completion of the addition, the mixture was further stirred for 10 minutes. The reaction solution was added dropwise into ice water and filtered. The filter cake was rinsed with 200 mL of water, and dried under reduced pressure to obtain 7.20 g of the title compound as a light brown solid, yield: 68.3%.

### Step 3: Preparation of 4-amino-5-hydroxy-2-nitrobenzoic acid (3c)

5-Methoxy-2-nitro-4-(2,2,2-trifluoroacetamido)benzoic acid (3b) (7.00 g, 22.7 mmol) was dissolved in 20% sodium hydroxide solution (50 mL) at room temperature, and the reaction solution was stirred under a nitrogen atmosphere at 100°C for 16 hours. The reaction solution was cooled to room temperature, to which concentrated hydrochloric acid (21 mL) was added dropwise under ice bath cooling. The reaction solution was concentrated under reduced pressure, to which anhydrous ethanol (420 mL) was added. The solution was stirred for 0.5 hour and filtered. The filtrate was concentrated under reduced pressure to obtain 7.20 g of the crude title compound as a brown solid, which was used directly in the next step without purification.

### Step 4: Preparation of methyl 4-amino-5-hydroxy-2-nitrobenzoate (3d)

4-Amino-5-hydroxy-2-nitrobenzoic acid (3c) (7.20 g, 36.4 mmol) was dissolved in MeOH (477 mL) at room temperature, to which dioxane in hydrochloric acid (161 mL) was added and stirred at 64°C for 24 hours. The reaction solution was concentrated under reduced pressure to obtain 6.20 g of the crude title compound as a black oily solid, which was used directly in the next step without purification.

### Step 5: Preparation of methyl 5-nitro-2-thioxo-2,3-dihydrobenzo[d]oxazole-6-carboxylate (3e)

Methyl 4-amino-5-hydroxy-2-nitrobenzoate (3d) (6.20 g, 29.2 mmol) was dissolved in ethanol (75 mL) at room temperature, to which carbon disulfide (24 mL) and potassium hydroxide (1.97 g, 35.1 mmol) were added. The reaction solution was stirred at 80°C for 16 hours, and concentrated under reduced pressure. The residues were purified by silica gel column chromatography (mobile phase: PE/EA = 10:1 to 3:1) to obtain 9.00 g of the crude title compound as a brown black solid, which was used directly in the next step without purification.

### Step 6: Preparation of methyl 2-morpholino-5-nitrobenzo[d]oxazole-6-carboxylate (3f)

Methyl 5-nitro-2-thioxo-2,3-dihydrobenzo[*d*]oxazole-6-carboxylate (3e) (9.00 g, 35.4 mmol) was dissolved in DMF (150 mL) at room temperature, to which morpholine (6.17 g, 70.9 mmol) and DIEA (9.21 g, 70.9 mmol) were added. The reaction solution was stirred at 80°C for 0.5 hour, and concentrated under reduced pressure. The residues were purified by silica gel column chromatography (mobile phase: PE/EA = 10:1 to 1:1) to obtain 600 mg of the title compound as a light yellow solid, yield: 5.52%.

### Step 7: Preparation of methyl 5-amino-2-morpholino-benzo[d]oxazole-6-carboxylate (3g)

Methyl 2-morpholino-5-nitrobenzo[*d*]oxazole-6-carboxylate (3f) (600 mg, 1.95 mmol) was dissolved in ethanol (20 mL) and water (2 mL) (ethanol : water = 10:1) at room temperature, to which iron powder (602 mg, 10.8 mmol) and ammonium chloride (73.2 mg, 1.37 mmol) were added. The reaction solution was stirred at 78°C for 4 hours, and extracted with 50 mL of ethyl acetate. The aqueous phase was extracted twice with 30 mL of ethyl acetate. The organic phases were combined, dried over anhydrous sodium sulfate and filtered. The filtrate was concentrated under reduced pressure. The residues were purified by silica gel column chromatography (mobile phase: PE/EA = 10:1 to 1:1) to obtain 400 mg of the title compound as a light yellow solid, yield: 74.1%.

### Step 8: Preparation of (5-amino-2-morpholino-benzo[d]oxazol-6-yl)methanol (3h)

Methyl 5-amino-2-morpholino-benzo[*d*]oxazole-6-carboxylate (3g) (800 mg, 3.88 mmol) was dissolved in THF (70 mL) at room temperature. LiAlH₄ (5.84 mL) was added dropwise under a nitrogen atmosphere at 0°C, and the reaction solution was stirred for 30 minutes. 10 mL of water was added to the reaction solution to quench the reaction. The reaction solution was extracted with ethyl acetate (80 mL), and the organic phase was concentrated under reduced pressure. The residues were purified by silica gel column chromatography (mobile phase: ethyl acetate) to obtain 350 mg of the title compound as a dark yellow solid, yield: 50.7%.

### Step 9: Preparation of 5-amino-2-morpholino-benzo[d]oxazole-6-carbaldehyde (3i)

(5-Amino-2-morpholino-benzo[*d*]oxazol-6-yl)methanol (3h) (340 mg, 1.91 mmol) was dissolved in DCM (10 mL) at room temperature. Manganese dioxide (3.32 g, 38.2 mmol) was added, and the reaction solution was stirred at room temperature for 4 hours. The reaction solution was filtered through celite, and the filtrate was concentrated under reduced pressure. The residues were purified by silica gel column chromatography (mobile phase: ethyl acetate) to obtain 200 mg of the title compound as a dark yellow solid, yield: 59.5%.

### Step 10: Preparation of 1-(3-ethoxy-2,3-dioxopropyl)pyridine-1-ammonium bromide (1b)

Pyridine (110 mg, 14.0 mmol) was dissolved in EtOH (10 mL) at room temperature, and then ethyl 3-bromo-2-oxopropanoate (246 mg, 1.26 mmol) was added under a nitrogen atmosphere. The reaction solution was stirred at 65°C for 2 hours, which was then used directly in the next step.

### Step 11: Preparation of 1-(6-(ethoxycarbonyl)-2-morpholinooxazolo[5,4-g]quinoline-7-yl)pyridine-1-ammoniu m bromide (3j)

The reaction solution of Step 10 was cooled to 18 to 22°C, followed by the addition of 5-amino-2-morpholino-benzo[*d*]oxazole-6-carbaldehyde (3i) (249 mg, 1.01 mmol) and pyridine (183 mg, 2.32 mmol). The reaction solution was stirred under a nitrogen atmosphere at 80°C for 9 hours, which was then used directly in the next step.

### Step 12: Preparation of ethyl 7-amino-2-morpholinooxazolo[5,4-g]quinoline-6-carboxylate (3k)

The reaction solution of Step 11 was cooled to 70°C, followed by the addition of morpholine (487 mg, 5.60 mmol). The reaction solution was warmed up to 80°C, and stirring under a nitrogen atmosphere at 80°C for 4 hours. 50 mL of water was added to the reaction solution which was then extracted with 50 mL of dichloromethane. The aqueous phase was extracted twice with 50 mL of dichloromethane. The organic phases were combined, dried over anhydrous sodium sulfate and filtered. The filtrate was concentrated under reduced pressure. The residues were purified by silica gel column chromatography (mobile phase: dichloromethane/ethyl acetate = 10:1 to 2:1) to obtain 210 mg of the title compound as a brown yellow solid, yield: 27.4%.

### Step 13: Preparation of 2-(7-amino-2-morpholinooxazolo[5,4-g]quinolin-6-yl)propan-2-ol (3)

Methylmagnesium chloride (3N, 0.97 mL, 2.92 mmol) was added dropwise to THF (10 mL) under a nitrogen atmosphere at 0°C. Ethyl 7-amino-2-morpholinooxazolo[5,4-*g*]quinoline-6-carboxylate (10k) (100 mg, 0.292 mmol) dissolved in THF (10 mL) was added, and the reaction solution was stirred at room temperature for 2 hours. 5 mL of water was added to quench the reaction. 15 mL of water was added to the reaction solution, and extracted with 40 mL of dichloromethane. The aqueous phase was extracted with 30 mL of dichloromethane. The organic phases were combined, dried over anhydrous sodium sulfate and filtered. The filtrate was concentrated under reduced pressure. The residues were purified by preparative liquid chromatography (column model: Daisogei 30 mm x 250 mm, C18, 10 um, 100 A, mobile phase: acetonitrile/water, gradient: 10%-50%) to obtain 8.00 mg of the title compound as a white solid, yield: 8.35%.

LC-MS: m/z 329.23 [M+H]⁺.

¹H NMR (300 MHz, DMSO-*d₆*) *δ* 7.52(s,1 H), 7.47 (s,1 H), 7.26 (s,1 H), 5.69 (s,3H), 3.75 ~ 3.72 (m,4 H), 3.65 ~ 3.62 (m,4 H), 1.61 (s, 6H).

### Example 4: Preparation of 2-(7-aminonaphtho[2,3-d][1,3]dioxol-6-yl)propan-2-ol

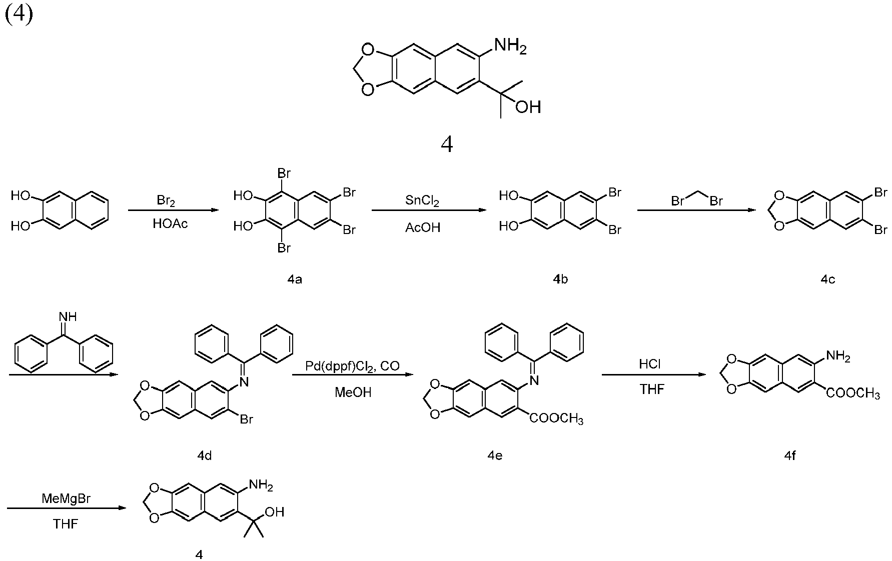

Step 1: Preparation of 1,4,6,7-tetrabromonaphthalene-2,3-diol (4a) Naphthalene-2,3-diol (3.00 g, 18.7 mmol) was added to acetic acid (30 mL) at room temperature, to which bromine (393 mg, 3.61 mmol) was added. The reaction solution was warmed up to 120°C and stirred for 45 minutes. The reaction solution was cooled to room temperature, and poured into ice water. The solution was extracted with ethyl acetate, and concentrated under reduced pressure. The residues were recrystallized from acetic acid to obtain 6.00 g of the title compound as a yellow solid, yield: 67.0%.

LC-MS: m/z 475.2 [M + H]⁺.

### Step 2: Preparation of 6,7-dibromonaphthalene-2,3-diol (4b)

1,4,6,7-Tetrabromonaphthalene-2,3-diol (4a) (500 mg, 1.10 mmol) was added to acetic acid (10 mL) at room temperature, followed by the addition of stannous chloride (2.00 g, 8.80 mmol). The reaction solution was warmed up to 120°C and stirred for 45 minutes. The reaction solution was cooled to room temperature, and concentrated under reduced pressure. The residues were recrystallized from toluene to obtain 300 mg of the crude title compound as a white solid, which was used directly in the next step without purification.

LC-MS: m/z 316.1 [M + H]⁺.

### Step 3: Preparation of 6,7-dibromonaphtho[2,3-d][1,3]dioxole (4c)

6,7-Dibromonaphthalene-2,3-diol (4b) (160 mg, 0.500 mmol) and dibromomethane (175 mg, 1.01 mmol) were added to *N*,*N*-dimethylformamide (10 mL) at room temperature, to which cesium carbonate (50.0 mg, 0.185 mmol) was added. The reaction solution was warmed up to 100°C and stirred for 50 minutes. The reaction solution was cooled to room temperature, and concentrated under reduced pressure. The residues were purified by silica gel column chromatography (eluent: EA/PE = 1:1) to obtain 20.0 mg of the title compound as a white solid, yield: 12.0%.

LC-MS: m/z 331 [M + H]⁺.

### Step 4: Preparation of N-(7-bromonaphtho[2,3-d][1,3]dioxol-6-yl)-1,1-diphenylmethanimine (4d)

6,7-Dibromonaphtho[2,3-*d*][1,3]dioxole (4c) (30.0 mg, 0.0900 mmol), dibenzylidimide (16.0 mg, 0.0900 mmol), sodium *tert*-butoxide (35.0 mg, 0.360 mmol), tris(dibenzylideneacetone)dipalladium (8.30 mg, 0.00900 mmol) and 4,5-bis(diphenylphosphine)-9,9-dimethylxanthene (11.0 mg, 0.0190 mmol) were added to anhydrous toluene (10 mL) at room temperature. The system was heated to 100°C under a nitrogen atmosphere and stirred for 1 hour. The reaction solution was cooled to room temperature and filtered through celite, and the filtrate was concentrated under reduced pressure. The residues were extracted with ethyl acetate, and washed with saturated sodium bicarbonate. The organic phase was dried over anhydrous sodium sulfate and filtered, and the filtrate was concentrated under reduced pressure. The residues were purified by silica gel column chromatography (eluent: PE/EA = 1:5) to obtain 50.0 mg of the title compound as a light yellow oil, yield: 64.0%.

LC-MS: m/z 431.6 [M + H]⁺.

### Step 5: Preparation of methyl 7-((diphenylmethylene)amino)naphtho[2,3-d][1,3]dioxole-6-carboxylate (4e)

*N*-(7-Bromonaphtho[2,3-*d*][1,3]dioxol-6-yl)-1,1-diphenylmethanimine (4d) (50.0 mg, 0.120 mmol), [1,1'-bis(diphenylphosphino)ferrocene]palladium dichloride (8.50 mg, 0.0110 mmol) and triethylamine (47.0 mg, 0.470 mmol) were added to methanol (5 mL) at room temperature. The system was heated to 120°C under a carbon monoxide atmosphere and stirred for 16 hours. The reaction solution was cooled to room temperature and filtered through celite, and the filtrate was concentrated under reduced pressure. The residues were extracted with ethyl acetate, and washed with saturated sodium bicarbonate. The organic phase was dried over anhydrous sodium sulfate and filtered, and the filtrate was concentrated under reduced pressure. The residues were purified by silica gel column chromatography (eluent: PE/EA = 1:2) to obtain 30.0 mg of the title compound as a light yellow oil, yield: 62.0%.

LC-MS: m/z 409.7 [M + H]⁺.

### Step 6: Preparation of methyl 7-aminonaphtho[2,3-d][1,3]dioxole-6-carboxylate (4f)

Methyl 7-((diphenylmethylene)amino)naphtho[2,3-*d*][1,3]dioxole-6-carboxylate (4e) (30.0 mg, 0.0700 mmol) was added to tetrahydrofuran (5 mL) and 3M hydrochloric acid (1 mL) at room temperature. The reaction system was stirred at room temperature for 16 hours, and concentrated under reduced pressure. The residues were extracted with ethyl acetate, and washed with saturated sodium bicarbonate. The organic phase was dried over anhydrous sodium sulfate and filtered, and the filtrate was concentrated under reduced pressure. The residues were purified by silica gel column chromatography (eluent: PE/EA = 2:1) to obtain 15.0 mg of the title compound as a light yellow oil, yield: 83.0%.

LC-MS: m/z 245.9 [M + H]⁺.

### Step 7: Preparation of 2-(7-aminonaphtho[2,3-d][1,3]dioxol-6-yl)propan-2-ol (4)

Methylmagnesium bromide (3 mol/L, 1.20 mL) was added to anhydrous tetrahydrofuran (10 mL) at room temperature. Methyl 7-aminonaphtho[2,3-*d*][1,3]dioxole-6-carboxylate (12f) (50 mg, 0.2 mmol) in anhydrous tetrahydrofuran (5 mL) was slowly added dropwise under a nitrogen atmosphere at 0°C. The reaction solution was stirred at 0°C for 1 hour. After completion of the reaction, the reaction solution was quenched with saturated aqueous ammonium chloride solution (10 mL), and extracted with ethyl acetate (40 mL). The organic phase was dried over anhydrous sodium sulfate and filtered, and the filtrate was concentrated under reduced pressure. The residues were purified by preparative liquid chromatography (column model: Gemini-C18 150 × 21.2 mm, 5um, mobile phase: acetonitrile/water, gradient: 10%-90%) to obtain 49.0 mg of the title compound as a white solid, yield: 50.1%.

LC-MS: m/z 246.1 [M + H]⁺.

¹H NMR (400 MHz, CD₃OD) *δ* 7.48 (s, 1H), 7.00 (s, 1H), 6.93 (s, 1H), 6.86 (s, 1H), 5.94 (s, 2H), 1.71(s, 6H).

### Example 5: Preparation of 2-(7-amino-3-methyl-3H-imidazo[4,5-g]quinolin-6-yl)propan-2-ol (5)

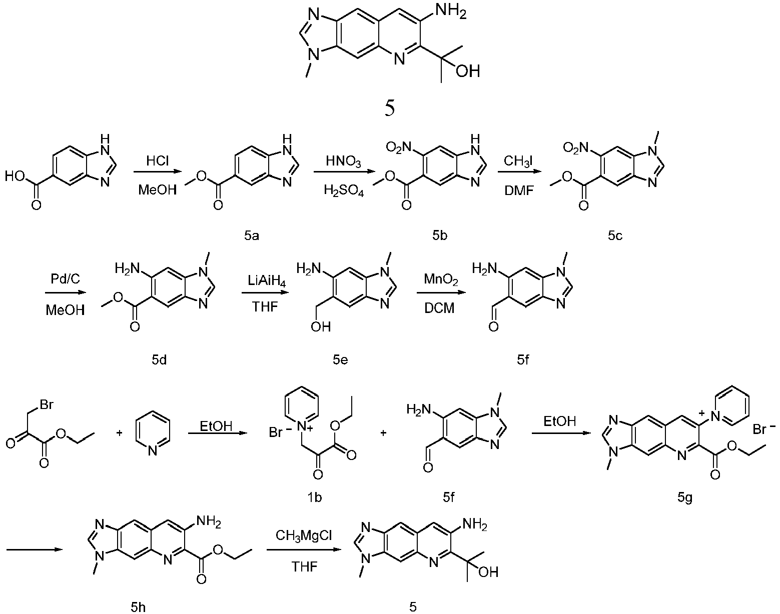

### Step 1: Preparation of methyl benzimidazole-5-carboxylate (5a)

Benzimidazole-5-carboxylic acid (5.00 g, 30.9 mmol) was dissolved in MeOH (30 mL), followed by the addition of HCl (30 mL, 4 N in dioxane) and stirring at 65°C overnight. The reaction solution was concentrated under reduced pressure and used directly in the next step.

### Step 2: Preparation of methyl 6-nitro-1H-benzo[d]imidazole-5-carboxylate (5b)

Concentrated sulfuric acid (100 mL) was added to concentrated nitric acid (100 mL) at 0°C, and methyl benzimidazole-5-carboxylate (5a) (5.00 g, 28.4 mmol) was added in batches. The reaction solution was stirring at room temperature overnight. The reaction solution was added dropwise to ice water and filtered. The filter cake was rinsed with 200 mL of water and dried under reduced pressure to obtain 5.00 g of the title compound as a yellow solid, yield: 79.6%.

### Step 3: Preparation of methyl 1-methyl-6-nitro-1H-benzo[d]imidazole-5-carboxylate (5c)

Methyl 6-nitro-1*H*-benzo[*d*]imidazole-5-carboxylate (5b) (5.00 g, 22.6 mmol), iodomethane (3.53 g, 24.8 mmol) and potassium carbonate (9.37 g, 67.9 mmol) were dissolved in DMF (100 mL) at room temperature and stirred overnight. 100 mL of water was added to the reaction solution which was then extracted with 100 mL of ethyl acetate. The aqueous phase was extracted twice with 100 mL of ethyl acetate. The organic phases were combined, dried over anhydrous sodium sulfate and filtered. The filtrate was concentrated under reduced pressure. The residues were purified by silica gel column chromatography (mobile phase: ethyl acetate) to obtain 3.00 g of the title compound as a yellow solid, yield: 56.4%.

### Step 4: Preparation of methyl 6-amino-1-methyl-1H-benzo[d]imidazole-5-carboxylate (5d)

Methyl 1-methyl-6-nitro-1*H*-benzo[*d*]imidazole-5-carboxylate (5c) (1.50 g, 6.38 mmol) was dissolved in MeOH (100 mL) at room temperature. Pd/C (200 mg) was added, and the reaction solution was stirred under a hydrogen atmosphere for 48 hours. The reaction solution was filtered through celite, and the filtrate was concentrated under reduced pressure. The residues were purified by silica gel column chromatography (mobile phase: dichloromethane/methanol = 40:1) to obtain 750 mg of the title compound as a yellow solid, yield: 57.3%.

### Step 5: Preparation of (6-amino-1-methyl-1H-benzo[d]imidazol-5-yl)methanol (5e)

Methyl 6-amino-1-methyl-1*H*-benzo[*d*]imidazole-5-carboxylate (5d) (650 mg, 3.17 mmol) was dissolved in THF (25 mL) at room temperature. LiAlH₄ (4.7 mL) was added dropwise under a nitrogen atmosphere at 0°C, and the reaction solution was stirred for 2 hours. 10 mL of methanol was added to the reaction solution, and the solution was concentrated under reduced pressure. The residues were purified by silica gel column chromatography (mobile phase: dichloromethane/methanol = 10:1) to obtain 450 mg of the title compound as a yellow solid, yield: 80.2%.

### Step 6: Preparation of 6-amino-1-methyl-1H-benzo[d]imidazole-5-carbaldehyde (5f)

(6-Amino-1-methyl-1*H*-benzo[*d*]imidazol-5-yl)methanol (5e) (450 mg, 2.54 mmol) was dissolved in DCM (20 mL) at room temperature. Manganese dioxide (4.42 g, 50.8 mmol) was added, and the reaction solution was stirred for 16 hours. The reaction solution was filtered through celite, and the filtrate was concentrated under reduced pressure. The residues were purified by silica gel column chromatography (mobile phase: dichloromethane/methanol = 20:1) to obtain 220 mg of the title compound as a yellow solid, yield: 49.4%.

### Step 7: Preparation of 1-(3-ethoxy-2,3-dioxopropyl)pyridine-1-ammonium bromide (1b)

Pyridine (70.8 mg, 0.896 mmol) was dissolved in EtOH (5 mL) at room temperature, and then ethyl 3-bromo-2-oxopropanoate (190 mg, 0.974 mmol) was added under a nitrogen atmosphere. The reaction solution was stirred at 65°C for 2 hours, which was then used directly in the next step.

### Step 8: Preparation of 1-(6-ethoxycarbonyl)-3-methyl-3H-imidazo[4,5-g]quinolin-7-yl)pyridin-1-ammonium bromide (5g)

The reaction solution of Step 7 was cooled to 18 to 22°C, followed by the addition of 6-amino-1-methyl-1*H*-benzo[*d*]imidazole-5-carbaldehyde (5f) (140 mg, 0.800 mmol) and pyridine (145 mg, 1.84 mmol). The reaction solution was stirred under a nitrogen atmosphere at 80°C overnight, which was then used directly in the next step.

### Step 9: Preparation of ethyl 7-amino-3 -methyl-3H-imidazo[4,5-g]quinoline-6-carboxylate (5h)

The reaction solution of Step 8 was cooled to 70°C, followed by the addition of morpholine (111 mg, 1.28 mmol). The reaction solution was warmed up to 80°C, and stirring under a nitrogen atmosphere at 80°C for 48 hours. 50 mL of water was added to the reaction solution which was then extracted with 50 mL of dichloromethane. The aqueous phase was extracted twice with 50 mL of dichloromethane. The organic phases were combined, dried over anhydrous sodium sulfate and filtered. The filtrate was concentrated under reduced pressure. The residues were purified by silica gel column chromatography (mobile phase: dichloromethane/methanol = 20:1) to obtain 110 mg of the title compound as a yellow solid, yield: 32.4%.

### Step 10: Preparation of 2-(7-amino-3-methyl-3H-imidazo[4,5-g]quinolin-6-yl)propan-2-ol (5)

Methylmagnesium chloride (3N, 0.8 mL, 2.40 mmol) was added dropwise to THF (5 mL) under a nitrogen atmosphere at 0°C. Ethyl 7-amino-3-methyl-3*H*-imidazo[4,5-*g*]quinoline-6-carboxylate (13h) (100 mg, 0.370 mmol) dissolved in THF (5 mL) was added, and the reaction solution was stirred at room temperature for 3 hours. 50 mL of water was added to the reaction solution, and extracted with 50 mL of dichloromethane. The aqueous phase was extracted twice with 50 mL of dichloromethane. The organic phases were combined, dried over anhydrous sodium sulfate and filtered. The filtrate was concentrated under reduced pressure. The residues were purified by preparative liquid chromatography (column model: Daisogei 30 mm × 250 mm, C18, 10 um, 100 A, mobile phase: acetonitrile/water (0.05% formic acid), gradient: 10%-60%) to obtain 44.25 mg of the title compound as a yellow solid, yield: 46.7%.

LC-MS: m/z 256.8 [M + H]⁺.

¹H NMR (400 MHz, CDCl₃) *δ* 8.33 (s,1 H), 7.97 (s, 1H), 8.04 (s, 1H), 7.41 (s, 1H), 3.85 (s, 3H), 1.76 (s, 6H).

### Example 6: Preparation of 2-(8-amino-2,3-dihydronaphtho[2,3-b][1,4]dioxin-7-yl)propan-2-ol (6)

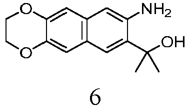

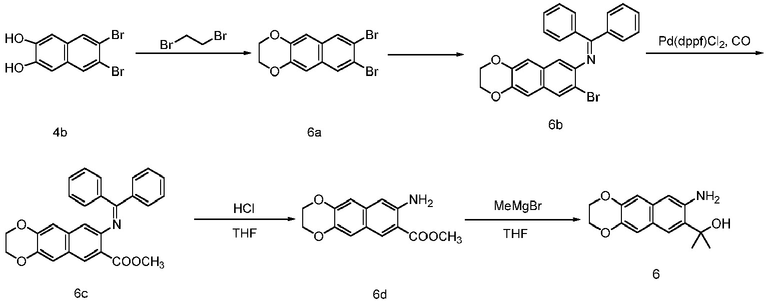

### Step 1: Preparation of 7,8-dibromo-2,3-dihydronaphtho[2,3-b][1,4]dioxine (6a)

6,7-Dibromonaphthalene-2,3-diol (4b) (1.00 g, 3.10 mmol) and potassium carbonate (1.75 g, 12.6 mmol) were added to acetonitrile (200 mL) at room temperature. The reaction solution was warmed up to 85°C and stirred for 10 minutes, to which dibromoethane (0.600 g, 3.10 mmol) was added dropwise and stirred for 1 hour. The reaction solution was cooled to room temperature, and concentrated under reduced pressure. The residues were purified by silica gel column chromatography (mobile phase: EA/PE = 3:1) to obtain 110 mg of the crude title compound as a yellow solid.

### Step 2: Preparation of N-(8-bromo-2,3-dihydronaphtho[2,3-b][1,4]dioxin-7-yl)-1,1-diphenylmethanimine (6b)

7,8-Dibromo-2,3-dihydronaphtho[2,3-*b*][1,4]dioxine (6a) (90.0 mg, 0.260 mmol), dibenzylidimide (47.0 mg, 0.260 mmol), sodium *tert*-butoxide (50.0 mg, 0.52 mmol), tris(dibenzylideneacetone)dipalladium (9.60 mg, 0.0100 mmol) and 4,5-bis(diphenylphosphine)-9,9-dimethylxanthene (12.0 mg, 0.0200 mmol) were added to anhydrous toluene (10 mL) at room temperature. The system was heated to 100°C under a nitrogen atmosphere and stirred for 1 hour. The reaction solution was cooled to room temperature and filtered through celite, and the filtrate was concentrated under reduced pressure. The residues were added to ethyl acetate and washed with saturated sodium bicarbonate. The organic phase was dried over anhydrous sodium sulfate, filtered and concentrated under reduced pressure. The residues were purified by silica gel column chromatography (mobile phase: EA/PE = 5:1) to obtain 29.0 mg of the crude title compound as a light yellow oil.

LC-MS: m/z 444.6 [M + H]⁺.

### Step 3: Preparation of methyl 8-((diphenylmethylene)amino)-2,3-dihydronaphtho[2,3-b][1,4]dioxine-7-carboxylate (6c)

*N*-(8-Bromo-2,3-dihydronaphtho[1,3-*b*][1,4]dioxin-7-yl)-1,1-diphenylmethanimine (6b) (29.0 mg, 0.0600 mmol), [1,1'-bis(diphenylphosphino)ferrocene]palladium dichloride (4.70 mg, 0.00600 mmol) and triethylamine (26.0 mg, 0.26 mmol) were added to methanol (5 mL) at room temperature. The system was then heated to 120°C under a carbon monoxide atmosphere and stirred for 16 hours. The reaction solution was cooled to room temperature and filtered through celite, and the filtrate was concentrated under reduced pressure. The residues were added to ethyl acetate, and washed with saturated sodium bicarbonate. The organic phase was dried over anhydrous sodium sulfate, filtered and concentrated under reduced pressure. The residues were purified by silica gel column chromatography (mobile phase: EA/PE = 2:1) to obtain 28.0 mg of the crude title compound as a light yellow oil.

LC-MS: m/z 424.1 [M + H]⁺.

### Step 4: Preparation of methyl 8-amino-1,3-dihydronaphtho[2,3-b][1,4]dioxine-7-carboxylate (6d)

Methyl 8-((diphenylmethylene)amino)-2,3-dihydronaphtho[2,3-*b*][1,4]dioxine-7-carboxylate (6c) (28.0 mg, 0.0700 mmol) was added to tetrahydrofuran (5 mL) and 3M hydrochloric acid (1 mL) at room temperature. The system was stirred at room temperature for 16 hours, and concentrated under reduced pressure. The residues were added to ethyl acetate and washed with saturated sodium bicarbonate. The organic phase was dried over anhydrous sodium sulfate, filtered and concentrated under reduced pressure. The residues were purified by silica gel column chromatography (mobile phase: EA/PE = 2:1) to obtain 25.0 mg of the crude title compound as a light yellow oil.

LC-MS: m/z 260.2 [M + H]⁺.

### Step 5: Preparation of 2-(8-amino-2,3-dihydronaphtho[2,3-b][1,4]dioxin-7-yl)propan-2-ol (6)

Methylmagnesium bromide (3 mol/L, 1.20 mL) was added to anhydrous tetrahydrofuran (10 mL) at room temperature. A solution of methyl 8-amino-1,3-dihydronaphtho[2,3-*b*][1,4]dioxine-7-carboxylate (19d) (48.0 mg, 0.190 mmol) in anhydrous tetrahydrofuran (5 mL) was slowly added dropwise under a nitrogen atmosphere at 0°C. The reaction solution was stirred at 0°C for 1 hour. After completion of the reaction, the reaction solution was quenched with saturated aqueous ammonium chloride solution (10 mL), and extracted with ethyl acetate (40 mL). The organic phase was dried over anhydrous sodium sulfate, filtered and concentrated under reduced pressure. The residues were purified by preparative liquid chromatography (column model: Gemini-C18 150 × 21.2 mm, 5um, mobile phase: acetonitrile/water, gradient: 10%-90%) to obtain 10.1 mg of the title compound as a white solid, yield: 21%.

LC-MS: m/z 259.1 [M + H]⁺.

¹H NMR (300 MHz, CD3OD) *δ* 7.42 (s, 1H), 7.05 (s, 1H), 6.89 (s, 1H), 6.84 (s, 1H), 4.25 (s, 4H), 1.69 (s, 6H).

### Example 7: Preparation of 2-(7-amino-2-morpholinooxazolo[4,5-g]quinolin-6-yl)propan-2-ol (7)

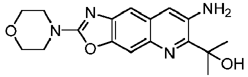

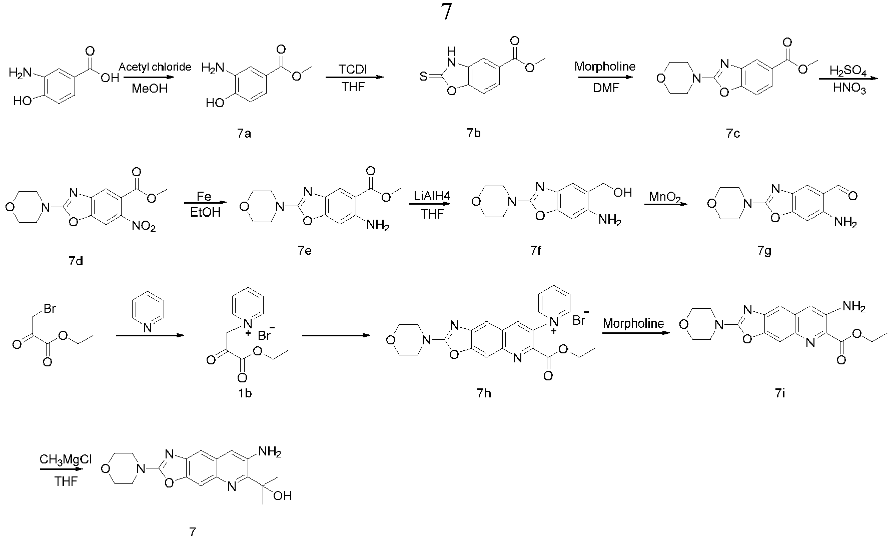

### Step 1: Preparation of methyl 3-amino-4-hydroxybenzoate (7a)

3-Amino-4-hydroxybenzoic acid (10.0 g, 65.4 mmol) was dissolved in methanol (500 mL) at room temperature, to which acetyl chloride (15.4 g, 196 mmol) was slowly added dropwise. The reaction solution was stirred at 72°C for 16 hours, and concentrated under reduced pressure to obtain 11.0 g of the crude title compound as a light green solid.

### Step 2: Preparation of methyl 2-thioxo-2,3-dihydrobenzo[d]oxazole-5-carboxylate (7b)

Methyl 3-amino-4-hydroxybenzoate (7a) (3.00 g, 18.0 mmol) was dissolved in TCDI (3.84 g, 21.6 mmol) at room temperature, and stirred at 30°C for 16 hours. A few drops of concentrated hydrochloric acid was added to the reaction solution to adjust it to weakly acidic. The solution was extracted with 60 mL of ethyl acetate, and the aqueous phase was extracted twice with 20 mL of ethyl acetate. The organic phases were combined, dried over anhydrous sodium sulfate and concentrated under reduced pressure to obtain 3.00 g of the crude title product as a light orange solid.

### Step 3: Preparation of methyl 2-morpholinobenzo[d]oxazole-5-carboxylate (7c)

Methyl 2-thioxo-2,3-dihydrobenzo[*d*]oxazole-5-carboxylate (7b) (3.00 g, 14.4 mmol) was dissolved in DMF (40 mL) at room temperature, to which morpholine (2.50 g, 28.7 mmol) and DIPEA (5.56 g, 43.1 mmol) were added. The reaction solution was stirred at 110°C for 2 hours. 30 mL of ice water was added to the reaction solution which was then extracted with 80 mL of ethyl acetate. The aqueous phase was extracted twice with 20 mL of ethyl acetate. The organic phases were combined, dried over anhydrous sodium sulfate and concentrated under reduced pressure. The residues were purified by silica gel column chromatography (mobile phase: PE/EA = 1:1) to obtain 3.00 g of the title product as a light yellow solid, yield: 79.5%.

### Step 4: Preparation of methyl 2-morpholino-6-nitrobenzo[d]oxazole-5-carboxylate (7d)

Methyl 2-morpholinobenzo[*d*]oxazole-5-carboxylate (7c) (2.00 g, 7.63 mmol) was dissolved in concentrated sulfuric acid (25 mL) at 0°C, to which concentrated nitric acid (15 mL) was slowly added dropwise. The reaction solution was stirred in an ice bath for 10 minutes. 30 mL of ice water was added to the reaction solution. The solution was extracted with 40 mL of ethyl acetate, and the aqueous phase was extracted twice with 20 mL of ethyl acetate. The organic phases were combined, dried over anhydrous sodium sulfate and filtered. The filtrate was concentrated under reduced pressure. The residues were purified by silica gel column chromatography (mobile phase: PE/EA = 2:1) to obtain 1.40 g of the title product as a yellow solid, yield: 59.8%.

### Step 5: Preparation of methyl 6-amino-2-morpholinobenzo[d]oxazole-5-carboxylate (7e)

Methyl 2-morpholino-6-nitrobenzo[*d*]oxazole-5-carboxylate (7d) (1.40 g, 4.56 mmol) was dissolved in ethanol (30 mL) and water (3 mL) (ethanol : water = 10:1) at room temperature, to which iron powder (1.41 g, 25.1 mmol) and ammonium chloride (171 mg, 3.19 mmol) were added. The reaction solution was stirred at 78°C for 16 hours. The reaction solution was extracted with 50 mL of ethyl acetate, and the aqueous phase was extracted twice with 20 mL of ethyl acetate. The organic phases were combined, washed with saturated sodium chloride solution, dried over anhydrous sodium sulfate and filtered. The filtrate was concentrated under reduced pressure. The residues were purified by silica gel column chromatography (mobile phase: DCM/EA = 1:1) to obtain 1.10 g of the title product as a light yellow solid, yield: 87.1%.

### Step 6: Preparation of (6-amino-2-morpholinobenzo[d]oxazol-5-yl)methanol (7f)

Methyl 6-amino-2-morpholinobenzo[*d*]oxazole-5-carboxylate (7e) (1.10 g, 3.97 mmol) was dissolved in THF (30 mL) at room temperature. LiAlH₄ (6.00 mL, 5.96 mmol) was added dropwise under a nitrogen atmosphere at 0°C, and the reaction solution was stirred in an ice bath for 1.5 hours. 5 mL of water was added to the reaction solution to quench the reaction. The solution was extracted with ethyl acetate (50 mL), and the aqueous phase was extracted twice with 30 mL of ethyl acetate. The organic phases were combined, dried over anhydrous sodium sulfate and concentrated under reduced pressure. The residues were purified by silica gel column chromatography (mobile phase: dichloromethane/methanol = 10:1) to obtain 800 mg of the title product as a brown solid, yield: 80.9%.

### Step 7: Preparation of 6-amino-2-morpholinobenzo[d]oxazole-5-carbaldehyde (7g)

(6-Amino-2-morpholinobenzo[*d*]oxazol-5-yl)methanol (7f) (550 mg, 2.21 mmol) was dissolved in DCM (45 mL) at room temperature. Manganese dioxide (3.84 g, 44.2 mmol) was added, and the reaction solution was stirred at room temperature for 16 hours. The reaction solution was filtered through celite, and the filtrate was concentrated under reduced pressure. The residues were purified by silica gel column chromatography (mobile phase: dichloromethane/methanol = 10:1) to obtain 400 mg of the title product as a brown solid, yield: 73.3%.

### Step 8: Preparation of 1-(3-ethoxy-2,3-dioxopropyl)pyridine-1-ammonium bromide (1b)

Pyridine (157 mg, 1.98 mmol) was dissolved in EtOH (30 mL) at room temperature, and then ethyl 3-bromo-2-oxopropanoate (351 mg, 1.80 mmol) was added under a nitrogen atmosphere. The reaction solution was stirred at 65°C for 2 hours, which was used directly in the next step.

### Step 9: Preparation of 1-(6-(ethoxycarbonyl)-2-morpholinooxazolo[4,5-g]quinoline-7-yl)pyridine-1 (7h)

The reaction solution of the above step was cooled to 18 to 22°C, followed by the addition of 6-amino-2-morpholinobenzo[*d*]oxazole-5-acetaldehyde (400 mg, 1.62 mmol) and pyridine (327 mg, 4.14 mmol). The reaction solution was stirred under a nitrogen atmosphere at 80°C for 16 hours, which was used directly in the next step.

### Step 10: Preparation of ethyl 7-amino-2-morpholinooxazolo[4,5-g]quinoline-6-carboxylate (7j)

The reaction solution of the previous step was cooled to 70°C, followed by the addition of morpholine (392 mg, 4.50 mmol). The reaction solution was warmed up to 80°C, and stirring under a nitrogen atmosphere for 4 hours. 30 mL of water was added to the reaction solution, and extracted with 40 mL of dichloromethane. The aqueous phase was extracted twice with 30 mL of dichloromethane. The organic phases were combined, dried over anhydrous sodium sulfate and filtered. The filtrate was concentrated under reduced pressure. The residues were purified by silica gel column chromatography (mobile phase: dichloromethane/ethyl acetate = 2:1) to obtain 126 mg of the title compound as a yellow solid, yield: 22.7%.

### Step 11: Preparation of 2-(7-amino-2-morpholinooxazolo[4,5-g]quinolin-6-yl)propan-2-ol (7)

Methylmagnesium chloride (3N, 0.78 mL, 2.34 mmol) was added dropwise to THF (10 mL) under a nitrogen atmosphere at 0°C, followed by the addition of ethyl 7-amino-2-morpholinooxazolo[4,5-*g*]quinoline-6-carboxylate (80 mg, 0.234 mmol) dissolved in THF (10 mL). The reaction solution was stirred at room temperature for 2 hours, and 5 mL of water was added to quench the reaction. 15 mL of water was added to the reaction solution which was then extracted with 30 mL of ethyl acetate. The aqueous phase was extracted twice with 30 mL of ethyl acetate. The organic phases were combined, dried over anhydrous sodium sulfate and filtered. The filtrate was concentrated under reduced pressure. The residues were purified by preparative liquid chromatography (column model: Daisogei 30 mm × 250 mm, C18, 10 um, 100 A, mobile phase: acetonitrile/water, gradient: 10%-50%) to obtain 16.0 mg of the title compound as a white solid, yield: 20.8%.

LC-MS: m/z 329.00 [M+H]⁺.

¹H NMR (300 MHz, DMSO-*d₆*) *δ* 7.59(s, 1 H), 7.31 (s, 1H), 7.22 (s, 1H), 5.69~5.66(s, 3H), 3.75 ~ 3.72 (m, 4H), 3.66 ~ 3.64 (m, 4H), 1.60 (s, 6H).

### Example 8: Preparation of 2-(14-amino-2,3,5,6,8,9-hexahydronaphtho[2,3-b][1,4,7,10]tetraoxacyclododecin-13-yl) propan-2-ol (8)

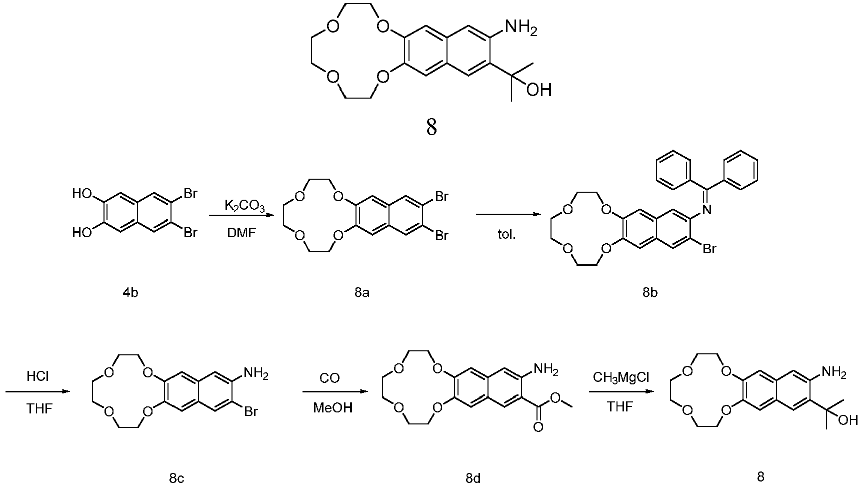

### Step 1: Preparation of 13,14-dibromo-2,3,5,6,8,9-hexahydronaphtho[2,3-b][1,4,7,10]tetraoxacyclododecine (8a)

6,7-Dibromonaphthalene-2,3-diol (4b) (1.60 g, 5.00 mmol) and 1,2-bis(2-bromoethoxy)ethane (9.25 g, 10.1 mmol) were added to N,N-dimethylformamide (50 mL) at room temperature. Potassium carbonate (2.77 g, 20.2 mmol) was added, and the reaction solution was warmed up to 120°C and stirred for 24 hours. The reaction solution was cooled to room temperature, and concentrated under reduced pressure. The residues were purified by silica gel column chromatography (eluent: EA/PE = 1:1) to obtain 590 mg of the title compound as a white solid, yield: 27.1%.

### Step 2: Preparation of N-(14-bromo-2,3,5,6,8,9-hexahydronaphtho[2,3-b][1,4,7,10]tetraoxacyclododecin-13-yl )-1,1-diphenylmethanimine (8b)

13,14-Dibromo-2,3,5,6,8,9-hexahydronaphtho[2,3-*b*][1,4,7,10]tetraoxacyclododeci ne (8a) (300 mg, 0.700 mmol), dibenzylidimide (139 mg, 0.735 mmol), sodium *tert*-butoxide (80.4 mg, 0.837 mmol), tris(dibenzylideneacetone)dipalladium (63.6 mg, 0.0696 mmol) and 1,1'-binaphthyl-2,2'-bis(diphenylphosphino) (64.8 mg, 0.105 mmol) were added to anhydrous toluene (30 mL) at room temperature. The system was heated to 80°C under a nitrogen atmosphere and stirred for 24 hours. The reaction solution was cooled to room temperature and filtered through celite, and the filtrate was concentrated under reduced pressure. The residues were added to ethyl acetate and washed with saturated sodium bicarbonate. The organic phase was dried over anhydrous sodium sulfate and filtered, and the filtrate was concentrated under reduced pressure. The residues were purified by silica gel column chromatography (eluent: EA) to obtain 360 mg of the title compound as a light yellow oil, yield: 97.1%.

LC-MS: m/z 532.1 [M + H]⁺.

### Step 3: Preparation of 14-bromo-2,3,5,6,8,9-hexahydronaphtho[2,3-b][1,4,7,10]tetraoxacyclododecin-13-amin e (8c)

*N*-(14-Bromo-2,3,5,6,8,9-hexahydronaphtho[2,3-*b*][1,4,7,10]tetraoxacyclododecin-13-yl)-1,1-diphenylmethanimine (8b) (300 mg, 0.565 mmol) was added to tetrahydrofuran (50 mL) and 3M hydrochloric acid (10 mL) at room temperature. The system was stirred at room temperature for 16 hours. The reaction solution was concentrated under reduced pressure, and the residues were added to ethyl acetate and washed with saturated sodium carbonate. The organic phase was dried over anhydrous sodium sulfate and filtered, and the filtrate was concentrated under reduced pressure. The residues were purified by silica gel column chromatography (eluent: EA) to obtain 150 mg of the title compound as a light yellow oil, yield: 72.3%.

LC-MS: m/z 368.2 [M + H]⁺.

### Step 4: Preparation of methyl 14-amino-2,3,5,6,8,9-hexahydronaphtho[2,3-b][1,4,7,10]tetraoxacyclododecine-13-carb oxylate (8d)

14-Bromo-2,3,5,6,8,9-hexahydronaphtho[2,3-*b*][1,4,7,10]tetraoxacyclododecin-13-amine (8c) (50.0 mg, 0.144 mmol), [1,1'-bis(diphenylphosphino)ferrocene]palladium dichloride (8.50 mg, 0.0144 mmol) and triethylamine (47.0 mg, 0.470 mmol) were added to methanol (5 mL) at room temperature. The system was heated to 120°C under a carbon monoxide atmosphere and stirred for 16 hours. The reaction solution was cooled to room temperature and filtered through celite, and the filtrate was concentrated under reduced pressure. The residues were extracted with ethyl acetate, and washed with saturated sodium bicarbonate. The organic phase was dried over anhydrous sodium sulfate and filtered, and the filtrate was concentrated under reduced pressure. The residues were purified by silica gel column chromatography (eluent: PE/EA = 1:2) to obtain 30.0 mg of the title compound as a light yellow oil, yield: 62.0%.

LC-MS: m/z 409.7 [M + H]⁺.

### Step 5: Preparation of 2-(14-amino-2,3,5,6,8,9-hexahydronaphtho[2,3-b][1,4,7,10]tetraoxacyclododecin-13-yl) propan-2-ol (8)

Methylmagnesium chloride (3 mol/L, 1.20 mL) was added to anhydrous tetrahydrofuran (10 mL) at room temperature. A solution of methyl 14-amino-2,3,5,6,8,9-hexahydronaphtho[2,3-*b*][1,4,7,10]tetraoxacyclododecine-13-carb oxylate (50 mg, 0.2 mmol) in anhydrous tetrahydrofuran (5 mL) was slowly added dropwise under a nitrogen atmosphere at 0°C. The reaction solution was stirred at 0°C for 1 hour. After completion of the reaction, the reaction solution was quenched with saturated aqueous ammonium chloride solution (10 mL), and extracted with ethyl acetate (40 mL). The organic phase was dried over anhydrous sodium sulfate and filtered, and the filtrate was concentrated under reduced pressure. The residues were purified by preparative liquid chromatography (column model: Gemini-C18 150 x 21.2 mm, 5um, mobile phase: acetonitrile/water, gradient: 10%-90%) to obtain 30.0 mg of the title compound as a white solid, yield: 60.0%.

LC-MS: m/z 330.0 [M - 17]⁺.

¹H NMR (400 MHz, DMSO) *δ* 7.65 (s, 1H), 7.31 (s, 1H), 6.98 (s, 1H), 6.78 (s, 1H), 5.54(s, 2H), 5.35 (s, 1H), 4.14-4.09 (m, 4H), 3.78-3.66 (m, 8H), 1.59(s, 6H).

### Example 9: Preparation of 1-(7-aminonaphtho[2,3-d][1,3]dioxol-6-yl)cyclopropan-1-ol (9)

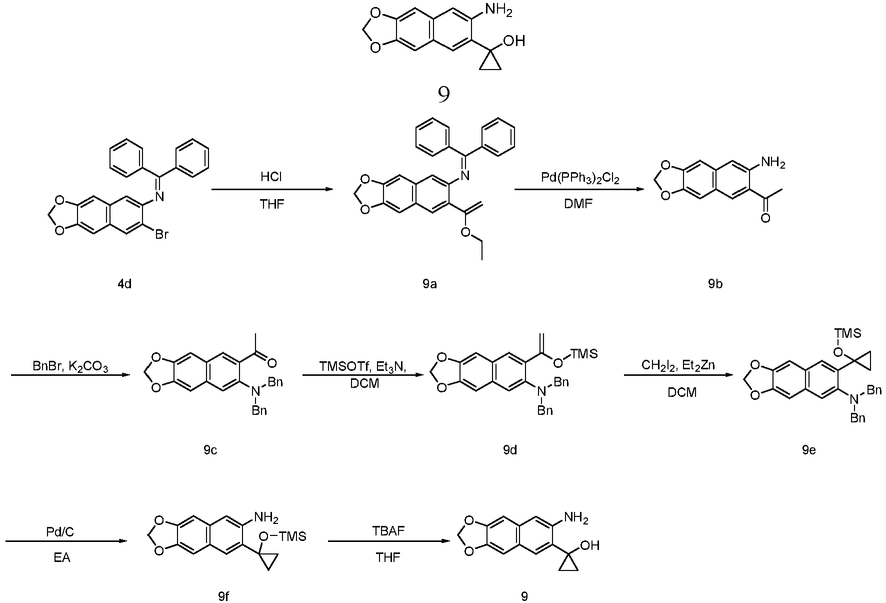

### Step 1: Preparation of N-(7-(1-ethoxyvinyl)naphtho[2,3-d][1,3]dioxol-6-yl)-1,1-diphenylmethanimine (9a)

*N*-(7-Bromonaphtho[2,3-*d*][1,3]dioxol-6-yl)-1,1-diphenylmethanimine (4d) (4.86 g, 11.3 mmol), tributyl(1-ethoxyvinyl)stannane (6.14 g, 17 mmol) and bis(triphenylphosphine)palladium chloride (795 mg, 1.13 mmol) were added to *N*,*N*-dimethylformamide (50 mL) at room temperature. The system was heated to 100°C under a nitrogen atmosphere and stirred for 18 hours. The reaction solution was cooled to room temperature, to which 100 mL of saturated potassium fluoride solution was added and stirred for 30 minutes. The reaction mixture was extracted with ethyl acetate and washed with saturated brine. The organic phase was dried over anhydrous sodium sulfate and filtered. The filtrate was concentrated under reduced pressure to obtain 4.77 g of the crude title compound as a dark brown oil.

LC-MS: m/z 422.1 [M + H]⁺.

### Step 2: Preparation of 1-(7-aminonaphtho[2,3-d][1,3]dioxol-6-yl)ethan-1-one (9b)

*N*-(7-(1-Ethoxyvinyl)naphtho[2,3-*d*][1,3]dioxol-6-yl)-1,1-diphenylmethanimine (9a) (4.77 g, 11.3 mmol) was dissolved in tetrahydrofuran (50 mL) at room temperature, followed by the addition of hydrochloric acid (3M, 50 mL). The reaction system was stirred at room temperature for 6 hours and concentrated under reduced pressure. The residues were adjusted to pH = 7-8 with saturated sodium carbonate solution. The resulting solid was filtered, rinsed and dried to obtain 2.09 g of the title compound as a light yellow solid, yield: 80.1%.

LC-MS: m/z 230.0 [M + H]⁺.

### Step 3: Preparation of 1-(7-(dibenzylamino)naphtho[2,3-d][1,3]dioxol-6-yl)ethan-1-one (9c)

1-(7-Aminonaphtho[2,3-*d*][1,3]dioxol-6-yl)ethan-1-one (9b) (1.00 g, 4.37 mmol), benzyl bromide (2.24 g, 13.1 mmol) and potassium carbonate (3.02 g, 21.85 mmol) were added to acetonitrile (30 mL) at room temperature. The system was stirred at 80°C for 10 hours and concentrated under reduced pressure. The residues were purified by silica gel column chromatography (mobile phase: PE/DCM = 1:1) to obtain 1.16 g of a yellow solid, yield: 64.8%.

LC-MS: m/z 410.1 [M + H]⁺.

### Step 4: Preparation of N,N-dibenzyl-7-(1-((trimethylsilyl)oxy)vinyl)naphtho[2,3-d][1,3]dioxol-6-amine (9d)

1-(7-(Dibenzylamino)naphtho[2,3-*d*][1,3]dioxol-6-yl)ethan-1-one (9c) (500 mg, 1.22 mmol) and triethylamine (370 mg, 3.67 mmol) were added to dichloromethane (8 mL) at room temperature. The mixture was cooled to 0°C, and trimethylsilyl trifluoromethanesulfonic acid (489 mg, 2.20 mmol) was added dropwise. The reaction system was stirred at room temperature for 18 hours, and then quenched by saturated sodium bicarbonate solution and extracted with dichloromethane. The organic phase was dried over anhydrous sodium sulfate, filtered and concentrated under reduced pressure to obtain 588 mg of the crude title compound as a yellow oil, which was used directly in the next step.

### Step 5: Preparation of N,N-dibenzyl-7-(1-((trimethylsilyl)oxy)cyclopropyl)naphtho[2,3-d][1,3]dioxol-6-amine (9e)

*N*,*N-*Dibenzyl-7-(1-((trimethylsilyl)oxy)vinyl)naphtho[2,3-*d*][1,3]dioxol-6-amine (9d) (588 mg, 1.222 mmol) and diiodomethane (655 mg, 2.444 mmol) were added to dichloromethane (10 mL) at room temperature. The mixture was cooled to 0°C, and diethylzinc (1M, 1.833 mL, 1.833 mmol) was added dropwise. The reaction system was stirred at room temperature for 18 hours, and then quenched by saturated ammonium chloride solution and extracted with dichloromethane. The organic phase was dried over anhydrous sodium sulfate and filtered, and the filtrate was concentrated under reduced pressure. The residues were purified by silica gel column chromatography (mobile phase: PE/DCM = 1:1) to obtain 30 mg of the title compound. Yield: 5%.

LC-MS: m/z 495.8 [M + H]⁺.

### Step 6: Preparation of 7-(1-((trimethylsilyl)oxy)cyclopropyl)naphtho[2,3-d][1,3]dioxol-6-amine (9f)

*N*,*N*-Dibenzyl-7-(1-((trimethylsilyl)oxy)cyclopropyl)naphtho[2,3-*d*][1,3]dioxol-6-a mine (9e) (120 mg, 0.242 mmol) and Pd/C (120 mg) were added to ethyl acetate (6 mL) at room temperature. The reaction solution was stirred under a hydrogen atmosphere at room temperature for 48 hours. The reaction solution was filtered and concentrated under reduced pressure. The residues were purified by silica gel column chromatography (mobile phase: PE/EA = 10:1) to obtain 21.0 mg of the title compound as a light yellow solid, yield: 27.5%.

LC-MS: m/z 316.0 [M + H]⁺.

### Step 7: Preparation of 1-(7-aminonaphtho[2,3-d][1,3]dioxol-6-yl)cyclopropan-1-ol (9)

7-(1-((Trimethylsilyl)oxy)cyclopropyl)naphtho[2,3-*d*][1,3]dioxol-6-amine (21.0 mg, 0.0670 mmol) was added to anhydrous tetrahydrofuran (3 mL) at room temperature. Tetrabutylammonium fluoride (1 M, 0.2 mL, 0.200 mmol) was slowly added dropwise under a nitrogen atmosphere, and the reaction solution was stirred at room temperature for 5 minutes. After completion of the reaction, ethyl acetate was added to the reaction solution, and the solution was washed twice with saturated sodium chloride solution. The organic phase was dried over anhydrous sodium sulfate and filtered, and the filtrate was concentrated under reduced pressure. The residues were purified by preparative liquid chromatography (column model: Gemini-C18 150 × 21.2 mm, 5 µm, mobile phase: acetonitrile/water, gradient: 20%-55%) to obtain 9.00 mg of the title compound as a yellow solid, yield: 55.3%.

LC-MS: m/z 244.1 [M + H]⁺.

¹H NMR (400 MHz, DMSO-*d₆*) *δ* 7.40 (s, 1H), 7.08 (s, 1H), 6.94 (s, 1H), 6.82 (s, 1H), 5.99 (s, 2H), 5.81 (s, 1H), 5.14 (s, 2H), 0.96-0.94 (m, 2H), 0.84-0.81 (m, 2H).

### Biological Assay

### Test Example 1: Test of the determination of aldehyde capture rate

In the aldehyde capture test disclosed in the present invention, the stable lipid metabolite, nonenal, was used as the model aldehyde to react with the compound of the Example. The specific protocol was as follows.

0.05 mM of the compounds of the Examples of the present invention were precisely weighed respectively and dissolved in 1.2 mL of a mixed solution (v/v=1:1) of triolein (Aladdin, G105172-5g) and linoleic acid (Aladdin, L100442-25g), respectively. 2.5 mL of PBS (Solarbio) solution containing 20% Captisol^{®} (Aladdin, C125030-25g) was added, and the solution was stirred at room temperature overnight. After the example compound was completely dissolved, 0.025 mM nonenal (Sigma, 255653-5G) was added, and the solution was stirred vigorously at room temperature. 200 µL of the sample was added to 800 µL of acetonitrile (Fisher, A995) at 0, 45, 90, 180, 225 and 465 minutes, respectively. The solution was mixed vigorously by vortex for 2 minutes, and centrifuged at a low speed of 1000 r/min for 3 minutes. The supernatant was transferred to an injection vial. The nonenal in the reaction mixture was analyzed quantitatively by high performance liquid chromatography. The adduct resulted from the example compound of the present invention and nonenal was analyzed qualitatively by high performance liquid chromatography-mass spectrometry.
Liquid chromatograph: Waters I Class;
Column: ACQUITY UPLC HSS T3 1.8µm;
Column temperature: 40°C;
Liquid chromatography condition: mobile phase A: acetonitrile containing 0.1% formic acid; B: water containing 0.1% formic acid;
Flow rate: 0.4 mL/mL;
Injection volume: 0.5 mL.
Gradient of the mobile phases:

| Time (min) | A% |
|---|---|
| 0 | 10 |
| 0.5 | 10 |
| 2.5 | 95 |
| 3.4 | 95 |
| 3.5 | 10 |
| 4.0 | 10 |

In the developed detection method, the retention time of the model aldehyde nonenal was 2.42 minutes.

Mass spectrometry conditions:
Mass spectrometer: Waters TQ-S micro
Ion source: ESI⁺
Capillary voltage: 0.5kV
Cone voltage: 10V
Source temperature: 150°C
Degassing temperature: 500°C, degassing flow: 1000L/Hr
SIM mode
m/z: 247.30 [M _{Example 1} + H]⁺, 369.30 [M _{adduct 1}+H]⁺

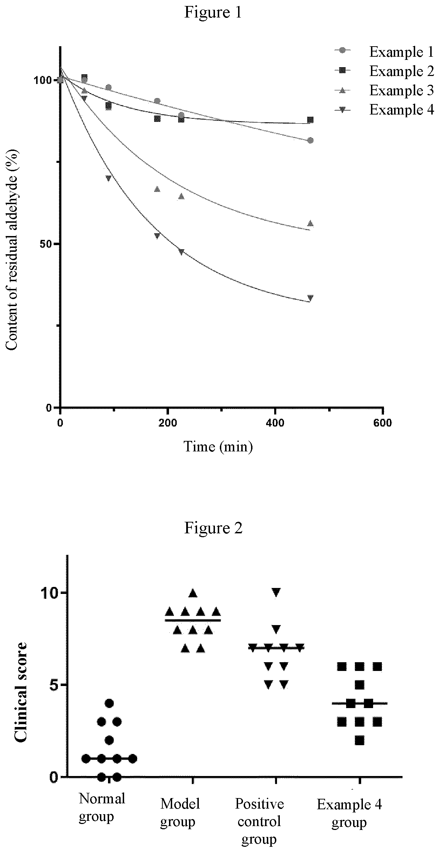

The variation of nonenal and some example compounds over reaction time is shown in Figure 1. The aldehyde consumption behavior of the example compounds of the present invention is shown in Table 1 below.

**Table 1 Aldehyde consumption behavior of the example compounds of the present invention at different time points**

| Examples | 225 minutes | | 465 minutes | |
|---|---|---|---|---|
| | Aldehyde consumption (100%) | Capture rate **^{a}** | Aldehyde consumption (100%) | Capture rate **^{a}** |
| Example 1 | 10.73 | -0.05 | 18.43 | -0.04 |
| Example 2 | 11.99 | -0.06 | 12.11 | -0.03 |
| Example 3 | 35.38 | -0.17 | 43.67 | -0.10 |
| Example 4 | 52.65 | -0.25 | 66.62 | -0.14 |
| Example 5 | 20.02 | -0.08 | 36.01 | -0.08 |
| Example 6 | 16.48 | -0.07 | 22.01 | -0.05 |
| Example 7 | 7.12 | -0.03 | 8.34 | -0.01 |
| Example 8 | 19.78 | -0.07 | 19.82 | -0.04 |
| Example 9 | 15.43 | -0.06 | 16.60 | -0.03 |

| | | | | |
|---|---|---|---|---|
| a. Aldehyde capture rate is represented by the slope of the capture curve per unit time, and the higher the absolute value of the slope, the higher the capture rate. | | | | |

It can be seen from Figure 1 and the above Table 1 that the compounds of the present invention have aldehyde capture ability.

### Test Example 2: Therapeutic effect of the compound of the present invention in uveitis animal model

Female lewis rats were used as the research subjects. Lewis rats (Vital River) were randomly divided into groups (4 animals per group, a total of 8 eyes). The groups are as follows: normal control group, model control group, and example administration group. The inflammation model was established in these groups except for the normal control group.

Formulation of eye drops:
(1) Formulation of drug vehicle stock solution: 2090 mg of Na₂HPO₄·12H₂O (Xilong Scientific, 9009012-01-09), 19 mg of NaH₂PO₄·2H₂O (Xilong Scientific, 9009013-01-09) and 9500 mg of β-cyclodextrin (Sigma, E1930253) were precisely weighed and dissolved in 30 mL sterile water for injection (Beijing CR Double-Crane), followed by the addition of 10 mL of PEG-400 (Solarbio, 222U011). The solution was mixed well, and made up to 50 mL with water for injection.
(2) Formulation of drug solution: 10 mg of the compound of Example 4 of the present invention was weighed and dissolved in 1 mL of the vehicle stock solution, and the resulting solution was made up to 2 mL with water for injection. The content of each substance is as follows: Na₂HPO₄·12H₂O 0.83%, NaH₂PO₄·2H₂O 0.017%, β-cyclodextrin 9.5%, PEG-400 5.0%, active compound 0.5%.
(3) The pH value of the formulated drug solution was determined by pH test paper. If the pH value was not within 7.3 ± 0.05, then it was adjusted to this range with 1 N HCl or 1 N NaOH. The solution was filtered through a 0.22 µm sterile filter (Merck, Millex), and stored at 4°C.

Handling of the animal model:
The animal model was a noninfectious uveitis animal model established by intraperitoneal injection of lipopolysaccharide (derived from *Escherichia coli,* 055:B5, Sigma, L2880-100MG, injection dose: 2 mg/kg). At the same time of modeling, drug administration was carried out according to the above grouping situation. The normal control group was administered with normal saline, the model control group was administered with blank vehicle, and the example administration group was administered with the eye drops formulated above. The drug was administered by eye instillation to both eyes of each animal, and the eye instillation dose was 20 µL/eye. After the completion of eye instillation, the eyelids were closed for 20 seconds to prevent the loss of the drug. The administration was repeated at 3, 6 and 23 hours after modeling. When the illness was severe (24 hours after modeling), the animals were anesthetized by intraperitoneal injection of 3.5% chloral hydrate. The ocular inflammation of the animals in each group was examined with a slit lamp microscope (Jiangxi Jiangfeng, LYL-S), and evaluated according to the McDonald-Shadduck scoring system (GB/T 28538-2012).

The animals were sacrificed, and the bilateral eyeballs were enucleated. The eyeball was subjected to corneal puncture. The aqueous humor of each eyeball was collected by a capillary, placed in a 1.5 mL centrifuge tube, and centrifuged at 1000 r/min. Quantitative analysis of total protein in aqueous humor was performed on the supernatant by a BCA protein quantification kit (Beyotime, P0010).

The effect of the compound of Example 4 of the present invention on the ocular inflammation score and protein concentration in aqueous humor of the ocular inflammation model rats are shown in Table 2 below.

**Table 2 Ocular inflammation score and protein concentration in aqueous humor of the model rats after treatment (mean ± standard deviation)**

| Group of the animals | Ocular score | Protein concentration in aqueous humor (µg/mL) |
|---|---|---|
| Normal control group | 4.5±1.4 | 623.8±83.3 |
| Model control group | 23.0±2.8 | 1664.1±215.6 |
| Example 4 group | 8.4±2.8*** | 840.6±449.7*** |

| | | |
|---|---|---|
| ^{∗∗∗}P<0.001, V.S. model control group | | |

It can be seen from the above Table 2 that in the ocular inflammation animal model, the treatment with the compound of Example 4 of the present invention can effectively reduce the ocular inflammation model score and significantly reduce the protein concentration in the aqueous humor, showing a therapeutic effect.

### Test Example 3: Therapeutic effect of the compound of the present invention on allergic conjunctivitis animal model

C48/80 is a polymer formed by the condensation of N-methyl-*p*-methoxyphenethylamine with formaldehyde, which acts directly on G protein and induces mast cell degranulation. After degranulation, the mast cell releases active substances such as histamine and kinin, which can cause acute type I allergic reactions such as telangiectasia and enhanced permeability. It can cause allergic conjunctivitis if applied topically to the ocular surface.

Female Wistar rats were used as the research subjects. Wistar rats (Vital River) were randomly divided into groups (5 animals per group, a total of 10 eyes). The groups are as follows: normal control group, model control group, positive drug group, and example administration group. The inflammation model was established in these groups except for the normal control group. Emedastine Difumarate Eye Drops (Emadine^{®}, Alcon, H20181192) was used as the positive drug. Model establishment, administration and evaluation processes are as follows.

The animal was subjected to inhalation anesthesia (isoflurane, Hebei Yipin pharmaceutical Co., Ltd., C002151205; anesthesia parameters: flow rate: 1.0 L/min, oxygen pressure: 0.1 MPa, solubility: 4.5%, anesthesia time: 5 minutes). The animal was grabbed with one hand and kept ventral side up. 10 µL of C48/80 solution (Sigma, C2313-100MG, 200 mg/mL, formulated with 0.9% saline) was added dropwise to the corneal surface of both eyes of the animal by a 10 µL pipette. The upper and lower eyelids were gently closed for 10 seconds to prevent the loss of the drug.

After 10 minutes of stimulation, different drug treatments were carried out according to the group settings. Both eyes of the same animal were subjected to the same drug treatment (10 µL per eye). The eyelids were closed for 10 seconds by the same method. Ophthalmological examination was performed 20 minutes after administration by a handheld slit lamp microscope (Jiangxi Jiangfeng, LYL-S). The clinical score was evaluated individually on the eyes of each animal according to the McDonald-Shadduck scoring system (GB/T 28538-2012).

The results are shown in Figure 2. According to the evaluation principle of the McDonald-Shadduck scoring system, the score value is positively correlated with the severity of ocular inflammation. The compound of Example 4 of the present invention has a good therapeutic effect on the allergic conjunctivitis in Wistar rats induced by C48/80.

## Claims

1. A compound of formula (I): 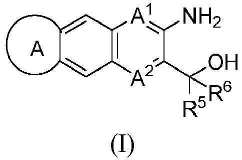
or a mesomer, racemate, enantiomer, diastereomer thereof, or mixture thereof, or a prodrug thereof, or a pharmaceutically acceptable salt thereof,
wherein:
A¹ is selected from the group consisting of N and CR¹;
A² is selected from the group consisting of N and CR²;
ring A is selected from the group consisting of 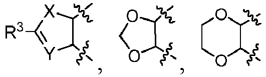 and 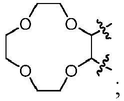
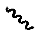 refers to the site attached to the phenyl moiety;
R¹ and R² are each independently selected from the group consisting of hydrogen, halogen, amino, nitro, cyano, hydroxy, thiol, oxo, alkyl, alkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
R³ is selected from the group consisting of hydrogen, halogen, amino, nitro, cyano, hydroxy, thiol, oxo, alkyl, alkoxy, haloalkyl, haloalkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
X and Y are each independently selected from the group consisting of O and NR⁴;
R⁴ is selected from the group consisting of hydrogen, halogen, amino, nitro, cyano, hydroxy, thiol, oxo, alkyl, alkoxy, haloalkyl, haloalkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
R⁵ and R⁶ are each independently selected from the group consisting of hydrogen, halogen, amino, cyano, hydroxy, thiol, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
or, R⁵ and R⁶ together with the carbon atom to which they are attached form a cycloalkyl or heterocyclyl, wherein the cycloalkyl or heterocyclyl is optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
or, R^{a} and R^{b} together with the nitrogen atom to which they are attached form a nitrogen-containing heterocyclyl, wherein the nitrogen-containing heterocyclyl is optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl and heteroaryl;
m is an integer from 0 to 2.

2. The compound of formula (I) according to claim 1, wherein:
A¹ is selected from CR¹; and
A² is selected from the group consisting of N and CR²;
R¹ and R² are each independently selected from the group consisting of hydrogen, halogen, C₁-C₆ alkyl, C₁-C₆ alkoxy, C₃-C₆ cycloalkyl and 5- to 6-membered heterocyclyl, and preferably hydrogen and C₁-C₆ alkyl.

3. The compound of formula (I) according to claim 1 or 2, wherein,
ring A is selected from the group consisting of 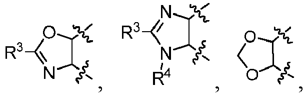 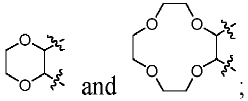
R³ is selected from the group consisting of hydrogen, halogen, amino, hydroxy, thiol, C₁-C₆ alkyl, C₁-C₆ alkoxy, C₁-C₆ haloalkyl, C₁-C₆ haloalkoxy, C₃-C₆ cycloalkyl, 5- to 6-membered heterocyclyl, C₆-C₁₀ aryl and 5- to 10-membered heteroaryl, and preferably hydrogen, C₁-C₆ alkyl, C₁-C₆ haloalkyl, C₃-C₆ cycloalkyl and 5- to 6-membered heterocyclyl;
R⁴ is selected from the group consisting of hydrogen, halogen, amino, hydroxy, thiol, C₁-C₆ alkyl, C₁-C₆ alkoxy, C₁-C₆ haloalkyl, C₁-C₆ haloalkoxy, C₃-C₆ cycloalkyl, 5- to 6-membered heterocyclyl, C₆-C₁₀ aryl and 5- to 10-membered heteroaryl, and preferably hydrogen, C₁-C₆ alkyl and C₁-C₆ haloalkyl.

4. The compound of formula (I) according to any one of claims 1 to 3,
wherein,
R⁵ and R⁶ are each independently selected from the group consisting of hydrogen, halogen, hydroxy, thiol, C₁-C₆ alkyl and C₃-C₆ cycloalkyl, and preferably hydroxy, thiol and C₁-C₆ alkyl, wherein the C₁-C₆ alkyl is optionally further substituted by one or more substituents selected from halogen;
or, R⁵ and R⁶ together with the carbon atom to which they are attached form a C₃-C₆ cycloalkyl, wherein the C₃-C₆ cycloalkyl is optionally further substituted by one or more substituents selected from the group consisting of halogen, hydroxy, thiol and C₁-C₆ alkyl.

5. The compound of formula (I) according to any one of claims 1 to 4, wherein the compound is selected from the group consisting of: 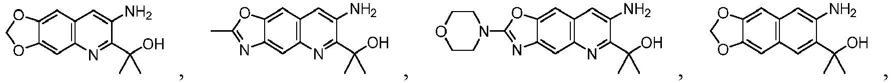 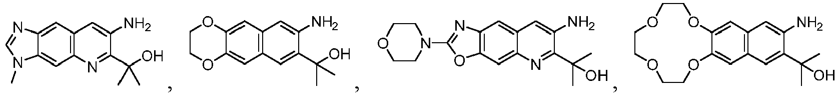 and

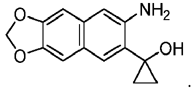

6. A method for preparing the compound of formula (I) according to any one of claims 1 to 5, comprising the following step of: 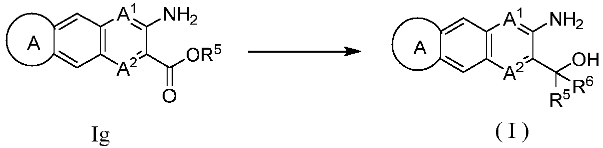
reacting compound Ig with an alkyl Grignard reagent to obtain the compound of formula (I), wherein the alkyl Grignard reagent is preferably methylmagnesium chloride or methylmagnesium bromide;
wherein A¹, A², ring A, R⁵ and R⁶ are as defined in claim 1.

7. A pharmaceutical composition comprising the compound of formula (I) according to any one of claims 1 to 5 or the pharmaceutically acceptable salt thereof and a pharmaceutically acceptable carrier.

8. Use of the compound of formula (I) according to any one of claims 1 to 5 or the pharmaceutically acceptable salt thereof, or the pharmaceutical composition according to claim 7 in the preparation of a toxic aldehyde capture agent.

9. Use of the compound of formula (I) according to any one of claims 1 to 5 or the pharmaceutically acceptable salt thereof, or the pharmaceutical composition according to claim 7 in the preparation of medicaments for the prevention and/or treatment of diseases related to active carbonyl compound; the disease is preferably ocular disease, skin disease, autoimmune disease, digestive system disease, cardiovascular disease, respiratory system disease, neurodegenerative disease, obesity, cancer, and aging-related disease; and the ocular disease is preferably noninfectious uveitis, allergic conjunctivitis and dry eye.
